# EUROPEAN PATENT APPLICATION

(11) **EP 3 396 383 A1**
(43) Date of publication of application: **31.10.2018**
(21) Application number: 18173015.1
(22) Date of filing: 10.09.2013
(51) Int. Cl.: G01N 33/68

(54) **ANALYSIS OF SALIVA PROTEOME FOR BIOMARKERS OF GINGIVITIS AND PERIODONTITIS USING FT-ICR-MS/MS**

(30) Priority: 10.09.2012 US 201261699035 P
(62) Divisional of application: 13792089.8
(71) Applicant: Koninklijke Philips N.V., 5656 AE Eindhoven (NL)
(72) Inventor: CHAPPLE, Iain, 5656 AE Eindhoven (NL); CREESE, Andrew, 5656 AE Eindhoven (NL); GRANT, Melissa, 5656 AE Eindhoven (NL)
(74) Representative: Coops, Peter

(57) **Abstract**

Methods for diagnosing the status of periodontitis disease includes selecting a set of protein biomarkers including one or more biomarkers which have been shown to vary in abundance at particular stages of periodontitis. The set of protein biomarkers may be identified and quantified in expression in an acquired gingival crevicular fluid (GCF) or saliva oral fluid sample in order to distinguish between different states of periodontitis. Methods of diagnosing the status of periodontitis oral disease at varying levels of severity, e.g. gingivitis, mild periodontitis, or severe periodontitis, may include selecting a set of protein biomarkers which are capable distinguishing between different stages of periodontitis.

## Description

The present application pertains to the fields of proteomics and bioinformatics. More particularly, the present application relates to diagnosing a status of an oral disease, e.g. periodontitis, at varying levels of severity through the quantification of protein biomarkers.

Gingivitis is a non-destructive form of periodontal disease involving soft tissue inflammation of the gums. Gingivitis typically occurs as a bodily response to bacterial biofilms, or plaques, which have adhered to teeth. In the absence of proper treatment, gingivitis may progress to periodontitis, which represents a destructive form of periodontal disease. Periodontitis may begin with a milder of the disease, which later progresses into severe periodontitis. Periodontitis is always preceded by the onset of gingivitis.

Periodontal diseases are the leading cause of tooth loss in adults. Accordingly, diagnostic tests have been developed to identify the probability of whether an individual has developed periodontitis. Oral-fluid-based point-of-case (POC) diagnostics are commonly used for various diagnostic tests in medicine and more recently are being adapted for the determination of oral diseases (Tabak, 2007, Ann N Y Acad Sci 1098: 7-14). The use of oral fluids for POC diagnostics has been shown to be effective in detecting oral cancer (Li et al., 2004, Clin Cancer Res 10:8442-8450; Zimmerman et al., 2008, Oral Oncol. 44(5):425-9) or HIV infection (Delaney et al., 2006, Aids 20: 1655-1660).

Periodontal diseases are presently diagnosed by evaluating clinical parameters such as pocket depth, bleeding on probing, and radiographs. These parameters have limitations in that they lack the ability to predict future attachment loss, and provide information only on the existence of past disease activity. Furthermore, no clinical parameters have been shown to be predictive for periodontal disease activity ("Clinical risk indicators for periodontal attachment loss," Journal of Clinical Periodontology 1991: v. 18:117-125"). Diagnostic methods in clinical practice today lack the ability to both detect the onset of inflammation, e.g. non-destructive gingivitis, and to identify the likelihood of developing destructive forms of periodontitis in the future.

Thus, there exists a need in the art for an efficient, accurate, and sensitive oral fluid diagnostic methods that can not only recognize the existence of past oral disease activity, but can also diagnose and assess earlier stages of oral diseases. In the case of periodontitis, oral fluid diagnostic methods should be able to distinguish at least between healthy patients and those that have developed gingivitis, milder forms of periodontitis, and/or more severe forms of periodontitis. This diagnostic method may advantageously include the quantification of particular protein biomarkers which are present in oral fluids. These oral fluids may be non-invasively acquired from a patient as gingival crevicular fluid (GCF) and/or saliva fluid.

### BRIEF SUMMARY

Demonstrated herein in an exemplary embodiment is a method for diagnosing the status of periodontitis disease. The method includes providing at least one of a gingival crevicular fluid (GCF) sample and a saliva sample, selecting a set of protein biomarkers for identifying a particular state of periodontitis, and determining the expression levels in the selected set of protein biomarkers to diagnose the status of periodontitis disease.

In an aspect of the method, the set of protein biomarkers is selected for distinguishing between a gingivitis state and a periodontitis state.

In another aspect of the method, the set of protein biomarkers is selected for distinguishing between a periodontal health and a disease state.

In yet another aspect of the method, the set of protein biomarkers is selected for distinguishing between a mild periodontitis state and a severe periodontitis state.

In some aspects of the method, the set of protein biomarkers includes at least one protein selected from the group consisting of haemoglobin chains alpha and beta, carbonic anhydrase 1(International Protein Index or "IPI" #IPI00980674), and plastin 1.

In another aspect of the method, the set of protein biomarkers includes at least one protein selected from the group consisting of S100-P, transaldolase, S100-A8 (calgranulin-A), myosin-9, Haemoglobin Alpha, and Haemoglobin Beta.

In yet another aspect of the method, the set of protein biomarkers includes at least one protein selected from the group consisting of Alpha-1-acid glycoprotein 1 and 2, matrix metalloproteinase-9, Peptidyl-prolyl cis-trans isomerase A, and Haptoglobin-related protein (IPI00431645.1).

In some aspect of the method, the set of protein biomarkers includes at least one protein selected from the group consisting of NADPH oxidase and Alpha-N-acetylgalactosaminidase.

In an aspect of the method, the set of protein biomarkers includes Alpha-N-acetylgalactosaminidase.

In another aspect of the method, the set of protein biomarkers includes at least one protein selected from the group consisting of Protein S100-A11 (IPI00013895.1), Protein IPI00037070.3, catalase (IPI00465436.4), Choline transporter-like protein 2 derivative (IPI00903245.1), and titin isoform N2-B (IPI00985334.2).

In yet another aspect of the method, the set of protein biomarkers includes two or more biomarkers.

In some aspects of the method, the method further includes providing both the GCF sample and saliva sample, generating a first and second protein profile by analyzing the proteome of a GCF sample and a saliva sample, and determining an overlap region between the first and second protein profiles. The set of protein biomarkers are selected for distinguishing between particular states of periodontitis, including calculating a change in abundance of proteins within the overlap region during different stages of periodontitis and selecting those proteins which are under or over expressed during a single state of periodontitis.

In another aspect of the method, the method further includes generating a protein profile by analyzing the proteome of the at least one oral fluid sample, and clustering the protein profile to determine a set of protein biomarkers.

Demonstrated herein in an exemplary embodiment is a kit for diagnosing the status of periodontitis disease. The kit includes a set of protein biomarkers selected to distinguish between gingivitis and periodontitis.

In an aspect of the kit, the set of protein biomarkers includes at least one protein selected from the group consisting of haemoglobin chains alpha and beta, carbonic anhydrase 1(International Protein Index or "IPI" #IPI00980674), and plastin-1.

In another aspect of the kit, the kit diagnoses gingivitis or mild periodontitis, and the set of protein biomarkers further includes at least one protein biomarkers from saliva data clusters 1B, 1D, 1A4, and 1A5.

### BRIEF DESCRIPTION OF THE DRAWINGS

These and other aspects, features and advantages of which the disclosed methods and kits are capable of will be apparent and elucidated from the following description of embodiments of the methods and kits, reference being made to the accompanying drawings, in which
FIG. 1 is a flow-chart illustration of a method for diagnosing a status of an oral disease according to one embodiment;
FIG. 2 is a graph of UV Absorbance (mAU) v. time (min) for a patient saliva sample. The UV trace was obtained as the output of an SCX system recording UV Absorbance at 214 nm.
FIG. 3 is a graph of UV Absorbance (mAU) v. time (min) for a patient GCF sample. The UV trace was obtained as the output of an SCX system recording UV Absorbance at 214 nm.
FIG. 4 is a group average clustering graph showing change in protein abundance (transformed by a base 2 logarithmic scale) vs. six (6) proteomic MS analysis groups (defined in TABLE 3) for GCF sample data. The group average clustering identified six (6) different clusters of protein biomarkers. Cluster 1 contained the majority of the proteins (243 proteins), cluster 2 contained 19 proteins, clusters 3, 5 and 6 each contained only one protein, and cluster 4 contained five proteins.
FIG. 5 is a first round re-clustering graph showing change in protein abundance (transformed by a base 2 logarithmic scale) vs. six (6) proteomic MS analysis groups (defined in TABLE 3) for GCF sample data. Cluster 1 from FIG. 4 was re-clustered into four cluster groups, where Group A contained the majority of the proteins (233), groups B and C contained two proteins each, and group D contained six proteins.
FIG. 6 is a second round clustering graph showing change in protein abundance (transformed by a base 2 logarithmic scale) vs. six (6) proteomic MS analysis groups (defined in TABLE 3) for GCF sample data. Cluster A from FIG. 5 was re-clustered into four cluster groups, where the largest cluster (1A1) still contained 171 proteins, cluster 1A2 contained 50 proteins, 1A3 contained 10 proteins, and 1A4 contained two proteins.
FIG. 7 is a final round clustering graph showing change in protein abundance (transformed by a base 2 logarithmic scale) vs. six (6) proteomic MS analysis groups (defined in TABLE 3) for GCF sample data. Cluster 1A1 from FIG. 6 was re-clustered into four groups. There are no clusters from this analysis which appear to be of interest, as the change in protein abundance is now below 1.0 in magnitude.
FIG. 8 is a group average clustering graph showing change in protein abundance (transformed by a base 2 logarithmic scale) vs. six (6) proteomic MS analysis groups (defined in TABLE 3) for Saliva sample data. The group average clustering identified five (5) different clusters of protein biomarkers. The largest cluster (1) contained 297 proteins, clusters 2 and 5 each contained one protein. Cluster 3 contained 11 proteins and cluster 4 contained three proteins. Cluster 2 appears to distinguish severe periodontitis from milder conditions.
FIG. 9 is a first round re-clustering graph showing change in protein abundance (transformed by a base 2 logarithmic scale) vs. six (6) proteomic MS analysis groups (defined in TABLE 3) for GCF sample data. Cluster 1 from FIG. 8 was re-clustered into four cluster groups, the largest of which contained 166 proteins (cluster 1A). Clusters IB and ID contained 14 and one protein respectively. These two groups may distinguish between gingivitis/mild periodontitis and severe periodontitis.
FIG. 10 is a second round re-clustering graph showing change in protein abundance (transformed by a base 2 logarithmic scale) vs. six (6) proteomic MS analysis groups (defined in TABLE 3) for GCF sample data. Cluster 1A from FIG. 9 was re-clustered into 5 cluster groups, the largest containing 150 proteins. There do not appear to be any significant clusters here based on lack of abundance change.
FIG. 11 is a second round re-clustering graph showing change in protein abundance (transformed by a base 2 logarithmic scale) vs. six (6) proteomic MS analysis groups (defined in TABLE 3) for GCF sample data. Cluster 1C from FIG. 9 was re-clustered into three clusters. Cluster 1C2 containing seven proteins show a near linear increase in proteins abundance up to severe periodontitis before a reduction post treatment.
FIG. 12 is a final round re-clustering graph showing change in protein abundance (transformed by a base 2 logarithmic scale) vs. six (6) proteomic MS analysis groups (defined in TABLE 3) for GCF sample data. Cluster 1A1 from FIG. 10. Five clusters were observed with a group of proteins showing an increase in abundance for severe periodontitis (cluster 1A1b) but none of the other groups. There were five proteins identified in this cluster.
FIG. 13 is a Venn diagram showing the overlap between the GCF and Saliva sample datasets.
FIG. 14 is a cluster graph showing Log (2) transformed abundance levels of protein S100-P vs six (6) proteomic MS analysis groups (defined in TABLE 3) in combined GCF and Saliva sample data.
FIG. 15 is a cluster graph showing Log (2) transformed abundance levels of protein S100-A8 vs six (6) proteomic MS analysis groups (defined in TABLE 3) in combined GCF and Saliva sample data.
FIG. 16 is a cluster graph showing Log (2) transformed abundance levels of myosin-9 vs six (6) proteomic MS analysis groups (defined in TABLE 3) in combined GCF and Saliva sample data.
FIG. 17 is a cluster graph showing Log (2) transformed abundance levels of transaldolase vs six (6) proteomic MS analysis groups (defined in TABLE 3) in combined GCF and Saliva sample data.
FIG. 18 is a cluster graph showing Log (2) transformed abundance levels of haemoglobin beta vs six (6) proteomic MS analysis groups (defined in TABLE 3) in combined GCF and Saliva sample data.

### DETAILED DESCRIPTION OF EMBODIMENTS

Several embodiments of the methods and kits of the present application will be described in more detail below with reference to the accompanying drawings in order for those skilled in the art to be able to carry out the disclosed methods and kits. The methods and kits may, however, be embodied in many different forms and should not be construed as limited to the embodiments set forth herein. Rather, these embodiments are provided so that this disclosure will be thorough and complete, and will fully convey the scope of the disclosed methods and kits to those skilled in the art. The embodiments do not limit the scope of disclosed methods or kits. The embodiments are only limited by the appended patent claims. Furthermore, the terminology used in the detailed description of the particular embodiments illustrated in the accompanying drawings is not intended to be limiting of the disclosed methods or kits.

The present application details methods for diagnosing the status of an oral disease, such as periodontitis. The methods may comprise determining the expression level of a set of biomarkers. The set of protein biomarkers may include one or more protein biomarkers which have been shown to vary in abundance at particular stages of oral disease. Accordingly, the set of protein biomarkers may be identified and quantified in expression in order to distinguish between different states of oral disease.

The methods of the present application demonstrate a role for biomarkers to serve as indicators of periodontitis at varying levels of severity, e.g. gingivitis, mild periodontitis. The work described herein demonstrates that elevated levels of multiple biomarkers can be used as a tool for accurately and rapidly determining the status of an oral disease, for example, periodontitis.

As used herein, the term "periodontal health state" is a threshold criteria based and not simply a vague state of health. Patients with a periodontal health state exhibit <10% sites with G.I. of 1.0 or B.O.P. and no sites with G.I. of 2.0 or 3.0. Additionally, they have no sites with interproximal attachment loss and no sites with ppd > 3mm.

As used herein, the term "gingivitis state" is a threshold criteria based on patients exhibiting generalized gingivitis and is not simply a vague state. Generalized gingivitis is shown in patients exhibiting > 30% of sites with G.I. > 2.0, no sites with interproximal attachment loss, and no sites with ppd > 4mm.

As used herein, the term "mild periodontitis state" is a threshold criteria based on patients exhibiting mild-moderate periodontitis and is not simply a vague state. Mild-moderate periodontitis is shown in patients exhibiting ppd of 5-7mm and interproximal CAL of 2-4mm at > 8 teeth).

As used herein, the term "severe periodontics state" is a threshold criteria based on patients exhibiting severe periodontitis and is not simply a vague state. Severe periodontitis is shown in patients exhibiting ppd of > 7mm and an interproximal CAL of > 5mm at > 12 teeth.

As used herein, the term "biomarker" means a substance that is measured objectively and evaluated as an indicator of normal biologic processes, pathogenic processes, or pharmacologic responses to a therapeutic intervention.

Provided herein is a method to diagnose a status of an oral disease by a measurement of prognostic protein biomarkers indicative of a select status of the oral disease. In an exemplary embodiment, the oral disease is periodontitis, and the protein biomarkers are indicative of either gingivitis, mild periodontitis, or severe periodontitis.

Through a proteomic analysis of gingival cervical fluid (GCF) and saliva samples taken from patients with varying states of oral disease, protein biomarkers may be identified which are increased or decreased in abundance during distinct phases of periodontitis. At least one protein biomarker may be used, alone or in combination, to distinguish between healthy patients, those suffering from gingivitis, and those suffering from mild or severe periodontitis.

Proteomic analysis may be conducted through a combination of liquid chromatography and mass spectrometry techniques. In particular, the proteome of GCF and Saliva oral liquid samples may be analyzed by Fourier Transform - tandem Mass Spectrometry (FT MS/MS). The FT MS/MS proteomic approach may be applied to GCF and Saliva samples collected from periodontally healthy volunteers, those with gingivitis, those with mild and severe periodontitis, and those with no teeth (edentulous controls), in order to try and elucidate a panel of biomarkers that will distinguish between healthy and diseased oral states. In particular, the FT MS/MS approach may be undertaken to discover novel protein biomarkers capable of distinguishing between periodontal health and disease, between gingivitis and periodontitis, and between mild and severe periodontitis, through the use of non-presumptive proteomic analysis of gingival crevicular fluid (GCF) and stimulated saliva.

The inventors have astonishingly found that the expression of a small set of particular protein biomarkers may be determined to identify gingivitis or mild periodontitis. These protein biomarkers show an enhanced change (either increase or decrease) in abundance during gingivitis/mild disease states of periodontitis, and show little changes during severe states of periodontitis. Additionally, the expression of a small set of particular protein biomarkers may be determined to identify severe periodontitis. These protein biomarkers show an enhanced change (either increase or decrease) in abundance during the severe state of periodontitis, and show little changes during gingivitis and mild states of periodontitis. The set of protein biomarkers for identifying and distinguishing severe gingivitis relative to mild periodontitis or gingivitis.

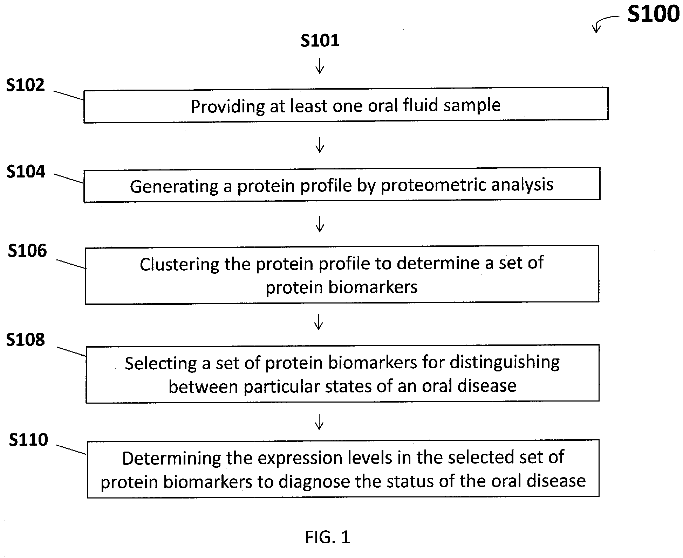

With reference to FIG. 1, a method **(S100)** for diagnosing a status of an oral disease starts at **S101.** According to an exemplary embodiment, the oral disease is periodontitis and the status of periodontitis may include periodontal health, gingivitis, mild periodontitis, and severe periodontitis.

At **S102,** at least one oral fluid sample is provided. According to one embodiment, the oral disease is periodontitis and at least one of a GCF and Saliva sample are provided. The samples may be non-invasively collected from a patient.

At **S104,** a protein profile is generated by analyzing the proteome of at least one of GCF and Saliva samples. In another embodiment, the protein profile is discovered using LC FT MS/MS.

At **S106,** the protein profile is clustered to determine those proteins which are best fit to serve in a set of protein biomarkers. Clustering may be performed using a combination of statistical methods including principle component analysis, gamma statistics, and metric multidimensional scaling (MMDS). In one embodiment, group average link hierarchical clustering is employed to determine the set of protein biomarkers. In another embodiment, complete link hierarchical clustering methods are employed to determine the set of protein biomarkers.

At **S108,** a set of protein biomarkers is selected for distinguishing between different states of an oral disease. In one embodiment, the oral disease is periodontitis and the set of protein biomarkers are selected for distinguishing between gingivitis and periodontitis. In another embodiment, the oral disease is periodontitis and the set of protein biomarkers are selected for distinguishing between mild periodontitis and severe periodontitis.

At **S110,** the expression levels of the proteins in the selected set of protein biomarkers are determined to diagnose the status of the oral disease.

According to one aspect of the methods, a method for diagnosing the status of an oral disease comprises providing at least one oral fluid sample, generating a protein profile by analyzing the proteome of the at least one oral fluid sample, clustering the protein profile to determine a set of protein biomarkers, selecting a set of protein biomarkers for distinguishing between particular states of an oral disease, and determining the expression levels in the selected set of protein biomarkers to diagnose the status of the oral disease.

According to yet another aspect of the methods, a method for diagnosing the status of periodontitis disease comprises providing at least one of a gingival crevicular fluid (GCF) and a saliva sample, selecting a set of protein biomarkers for identifying a particular state of periodontitis, and determining the expression levels in the selected set of protein biomarkers to diagnose the status of the oral disease.

In some aspect of the methods, the set of biomarkers is selected by analyzing the proteome of gingival crevicular fluid (GCF) and saliva. Proteomic analysis may include Fourier Transform - tandem Mass Spectrometry (FT MS/MS) analysis of proteins which are identified to be over or under expressed in varying states of periodontitis.

Another aspect of the methods, the biomarkers may include only one, or a combination of particular biomarkers which are useful for the diagnosis of a disease state. The expression levels of one, two, or more protein biomarkers are determined to determine a status of an oral disease. In further aspects, three, four, five, or more biomarkers are determined and used to determine the status of an oral disease.

In various aspects of the methods, the one or more protein biomarkers are selected from the group consisting of haemoglobin chains alpha and beta, carbonic anhydrase 1(International Protein Index or "IPI" #IPI00980674), and plastin 1. The method according to this aspect may be used to distinguish between a healthy state, gingivitis state, a mild state, and a severe state of periodontitis.

In yet another aspect of the methods, one or more protein biomarkers are selected from the group consisting of S100-P, transaldolase, S100-A8 (calgranulin-A), myosin-9, Haemoglobin Alpha, and Haemoglobin Beta. The method according to this aspect may be used to identify the severe state of periodontitis, and distinguish to the severe state of periodontitis from the milder states, e.g. mild periodontitis and gingivitis.

In some aspects of the disclosed methods, one or more protein biomarkers are selected from the group consisting of Alpha-1-acid glycoprotein 1 and 2, matrix metalloproteinase-9, Peptidyl-prolyl cis-trans isomerase A, and Haptoglobin-related protein (IPI00431645.1). The method according to this aspect may be used to identify the severe state of periodontitis, and distinguish to the severe state of periodontitis from the milder states, e.g. mild periodontitis and gingivitis.

In still another aspect of the methods, protein biomarker Alpha-N-acetylgalactosaminidase is selected for identifying gingivitis or mild periodontitis state, and distinguishing them from a severe periodontitis state.

In a further aspect of the methods, one or more protein biomarkers are selected from NADPH oxidase and Alpha-N-acetylgalactosaminidase for identifying gingivitis or mild periodontitis, and distinguishing them from a severe periodontitis state.

According to some aspects, the method for diagnosing the status of an oral disease further includes providing the GCF and saliva sample, generating a first and second protein profile by analyzing the proteome of a GCF sample and a saliva sample, and determining an overlap region between the first and second protein profiles. The selecting the set of protein biomarkers for distinguishing between particular states of periodontitis may include calculating a change in abundance of proteins within the overlap region during different stages of periodontitis and selecting those proteins which are under or over expressed during a single state of periodontitis.

In yet another aspect, the method for diagnosing a status of an oral disease as disclosed by the previous embodiments is performed by a diagnostic kit. The diagnostic kit comprises a set of protein biomarkers for identifying the status of an oral disease. The kit includes the necessary reagents to carry out the assays of the disclosed methods.

While the present application has been described in terms of various embodiments and examples, it is understood that variations or improvements will occur to those skilled in the art. Therefore, only such limitations as appear in the claims should be placed on the disclosed embodiments.

### EXAMPLES

### Example 1

The proteome of gingival crevicular fluid (GCF) and saliva was analyzed to identify biomarkers for different oral disease states, e.g. gingivitis, mild periodontitis, and severe periodontitis. GCF and saliva samples were collected non-invasively from the mouths of several patients. Liquid chromatography techniques coupled with Fourier Transform - tandem Mass Spectrometry (FT MS/MS) were used to separate protein biomarkers from within the samples and to identify the protein biomarkers.

The FT MS/MS proteomic approach was applied to samples collected from periodontally healthy volunteers, those with gingivitis, those with mild and severe periodontitis, and those with no teeth (edentulous controls), in order to try and elucidate a panel of biomarkers that will distinguish between healthy and diseased oral states. In particular, the FTMS/MS approach was undertaken to discover novel protein biomarkers capable of:
1. distinguishing between periodontal health and disease
2. distinguishing between gingivitis and periodontitis
3. distinguishing between mild and severe periodontitis
by non-presumptive proteomic analysis of gingival crevicular fluid (GCF) and stimulated saliva.

### STUDY DESIGN

**TABLE 1**

| **Patient Group Number For Sample Collection** | **Oral Disease State** |
|---|---|
| Group 1 | Patients with periodontal health (<10% sites with G.I. of 1.0 or B.O.P. & no sites with G.I. of 2.0 or 3.0. No sites with interproximal attachment loss and no sites with ppd > 3mm). |
| Group 2 | Patients with generalized gingivitis (>30% of sites with G.I. > 2.0, no sites with interproximal attachment loss & no sites with ppd >4mm). |
| Group 3 | Patients with mild-moderate periodontitis (ppd of 5-7mm and interproximal CAL of 2-4mm at > 8 teeth). |
| Group 4 | Patients with severe periodontitis (ppd of > 7mm & interproximal CAL of > 5mm at > 12 teeth). |
| Group 5 | Edentulous patients (no teeth) with no evidence of oral ulceration or erosive disease. |

Five groups of patient volunteers were recruited as defined in TABLE 1. The study was performed as a cross-sectional study with no interventions planned other than routine therapy that may be clinically indicated. Only 1 visit was required at baseline for sampling, but those in Group 3 and 4 required routine periodontal scaling, root surface debridement and prophylaxis and were therefore re-examined and sampled 3-months following completion of their therapy. Longitudinal analysis was therefore available for groups 3 and 4. The clinical assessments were carried out by a trained study dental surgeon.

### SAMPLE COLLECTION

Volunteers were asked to provide 6 samples of GCF collected from the gingival (gum) margin, non-invasively on standard filter papers strips (Periopapers™). They were also asked to provide a stimulated saliva sample by rolling a sterile marble around their mouths for five minutes and expectorating into a graduated sterile glass collection vile for volume measurement.

GCF and saliva were collected from 10 volunteers in each of five clearly defined phenotypic groups: healthy, gingivitis, mild periodontitis, severe periodontitis, and edentulous patients as a -ve control group. A total of 50 patients were therefore recruited and sampled. Volunteers with periodontitis (Groups 3 & 4) were then treated non-surgically in order to remove the periodontal inflammation and restore improved health. GCF and saliva were also collected 3 months post-treatment in these two groups, providing longitudinal data. Table 2 presents the mean clinical data at a time of baseline and post-therapy obtained from 50 patients representing five phenotypic groups. GCF and Saliva samples were collected from 10 volunteers in each of five clearly defined phenotypic groups: Group I (healthy), Group II (gingivitis), Group III (mild periodontitis), Group IV (severe periodontitis), Group V (edentulous patients as a negative control group), where the phenotypic groups are defined based on predefined clinical data thresholds. Volunteers with periodontitis (Groups 3 & 4) were treated non-surgically in order to remove the periodontal inflammation and restore improved health, and therefore have both "baseline" and "review" clinical data.

**TABLE 2**

| **Criteria** | **Time** | **Group 1** | **Group 2** | **Group 3** | **Group 4** | **Group 5** |
|---|---|---|---|---|---|---|
| **PPD** (mm) | Baseline | **1.31** + 0.65 | **1.89** + .070 | **3.35** + 1.67 | **4.68** + 2.33 | - |
| | Review | - | - | **2.45** + 1.06 | **3.06 +** 1.56 | - |
| **REC** (mm) | Baseline | **0.00** + 0.00 | **0.00** + 0.00 | **0.70** + 0.86 | **0.77** + 0.89 | - |
| | Review | - | - | **0.83** + .093 | **1.34** + 1.07 | - |
| **CAL** (mm) | Baseline | **1.31** + 0.65 | **1.89** + .070 | **4.05** + 1.88 | **5.45** + 2.49 | - |
| | Review | - | - | **3.27** + 1.44 | **4.37** + 1.92 | - |
| **BOP d** (% sites) | Baseline | **3.60** + 2.24 | - | **100.57** + 61.28 | **127.29** + 71.10 | - |
| | Review | - | - | **29.22** + 18.36 | **40.13** + 22.07 | - |
| **BOP m** (% sites) | Baseline | **2.80** +2.56 | **27.00** + 24.9 | **51.70** + 47.40 | **71.30** + 60.79 | - |
| | Review | - | - | **15.20** + 13.69 | **20.60** + 17.02 | - |
| **MGI** (FM total) | Baseline | **44.00** | **127.10** | **189.30** | **192.90** | - |
| | Review | - | - | **83.90** | **112.70** | - |
| **PI** (% sites) | Baseline | - | - | **79.80** + 72.72 | **86.90** + 73.73 | - |
| | Review | - | - | **55.67** + 50.60 | **49.80** + 42.38 | - |
| **GCF** (mean vol µls) | Baseline | **0.10** + 0.08 | **0.29** + 0.14 | **0.33** + 0.20 | **0.49** + 0.27 | - |
| | Review | - | - | **0.23** + 0.15 | **0.32** + 0.23 | - |

### SAMPLE PROCESSING

### GFC Samples

GCF samples were collected on periopaper strips from the mesio-buccal sites of six teeth per volunteer, for 30 seconds as is convention and volumes read on a Perotron 8000™ (Chapple *et al* 1999). These were placed in 400µL of a 100mM ammonium bicarbonate buffer in 1.5 mL screw top cryo-tubes. The GCF samples were immediately frozen to -80°C.

Prior to analysis GCF was defrosted on ice. The tubes were vortexed for 30 seconds and the solution removed into a clean snaptop eppendorf tube. 200µL of ammonium bicarbonate (100 mM) was added to the strips. These were re-vortexed for 30 seconds and re-centrifuged at 13,000 RPM for five minutes. The solution was removed and added to the previous. From each sample within a group 150µL was combined to give a single "pooled" sample per group. Individual patient samples were held back to allow a post-study re-evaluation at a patient-specific level once the preferred biomarker panels had been elucidated. Therefore 6 x 1.5 mL "population" samples were available for proteomic analysis by MS as indicated in Table 3.

**TABLE 3**

| **Group Number for MS Analysis** | **Oral Disease State** |
|---|---|
| Group 1 | Healthy |
| Group 2 | Gingivitis |
| Group 3 | Mild periodontitis pre-treatment |
| Group 4 | Severe periodontitis pre-treatment |
| Group 5 | Mild periodontitis post-treatment |
| Group 6 | Severe periodontitis post-treatment |

### Saliva Samples

Saliva production was stimulated using a sterile marble and collected for five minutes into 15 mL Falcon tubes. Tubes were frozen at -80°C. Prior to analysis the saliva was defrosted at 4°C. Additional falcon tubes were weighed prior to defrost to transfer the clarified saliva to. Once defrosted the saliva was aliquoted into 1.5 mL snaptop eppendorf tubes and centrifuged at 13,000 rpm for five minutes. The supernatant was transferred into the pre-weighed tubes. The debris pellet was also retained for potential future analysis. Both the weight and volume of saliva was recorded. 10.5µL of each saliva sample per group was combined in the same manner as GCF samples. However, unlike GCF, saliva was available from the edentulous patient group (Group 5), therefore a total of 7 x 105µL "population" saliva samples resulted. As for GCF the individual patient samples were held back to allow future "patient-level" analysis. The Pooled saliva samples were centrifuged at 13,000 RPM for five minutes and 100µL retained. This was to ensure no debris was transferred into the final sample. Ammonium bicarbonate (100µL, 200mM) was added to each sample.

### SAMPLE ANALYSIS BY LC FT MS/MS

Dithiothrietol was added (20µL, 50mM) to both GCF and saliva samples, which were incubated with shaking at 60 °C for 45 minutes to reduce any disulphide bonds. The samples were returned to room temperature prior to addition of iodoacetamide (100µL, 22mM) and incubation at room temperature in the dark for 25 minutes. Iodoacetamide alkylates free thiol group on cysteine residues. Dithiothrietol (2.8µL, 50mM) was added to quench any remaining iodoacetamide. 1 µg of Lys-C (cleaves proteins at the C terminus of lysine residues) was added to each sample (1:100 enzyme:protein) and incubated at 37°C with shaking for four hours. 2µg of trypsin (cleaves proteins at the C terminus of lysine and arginine residues) was added and the digest continued over night at 37°C.

The samples were vacuum centrifuged dry prior to desalting (required for iTRAQ labelling). The samples were acidified (200µL, 0.5% TFA) and desalting was performed using a Macrotrap (Michrom). The trap was wetted with acetonitrile (3x 50%, 200µL) followed by washing with trifluoroacetic acid (3x 0.1%, 200µL). The sample was then loaded through the trap and the elutant passed through the trap again. The trap was washed again with trifluoroacetic acid (3x 0.1%, 200µL), finally the peptides were eluted with acetonitrile (70%, 100µL). The samples were vacuum centrifuged dry.

The dry samples were labeled with the iTRAQ 8-plex labels as shown in Table 4 below. The labeling allows all samples to be subsequently mixed together and run under one set of conditions in triplicate. Subsequently the individual group samples were identified from the iTRAQ labels.

**TABLE 4**

| **ITRAQ LABELS** | **SALIVA DISEASE STATE** | **GCF DISEASE STATE** |
|---|---|---|
| 113 | Healthy | Healthy |
| 114 | Gingivitis | Gingivitis |
| 115 | Mild Periodontitis | Mild Periodontitis |
| 116 | Severe Periodontitis | Severe Periodontitis |
| 117 | Mild Periodontitis Treated | Mild Periodontitis Treated |
| 118 | Severe Periodontitis Treated | Severe Periodontitis Treated |
| 119 | Edentulous | |

The samples were incubated with the labels for two hours at room temperature before all individual samples were mixed together for GCF and Saliva respectively. The combined samples (1 pooled saliva and 1 pooled GCF) were vacuum centrifuged dry. The samples were re-suspended in 100µL of mobile phase A for the SCX system (10 mM KH₂PO₄, pH 3, 20% MeCN). The peptides were separated using strong cation exchange chromatography using the above mobile phase A and mobile phase B (10 mM KH₂PO₄, 500 mM KCl, pH 3, 20% MeCN). The gradient ran for 90 minutes. 15 fractions were collected. Fractions 15 and 12 were combined as were 13 and 14 to give 13 fractions.

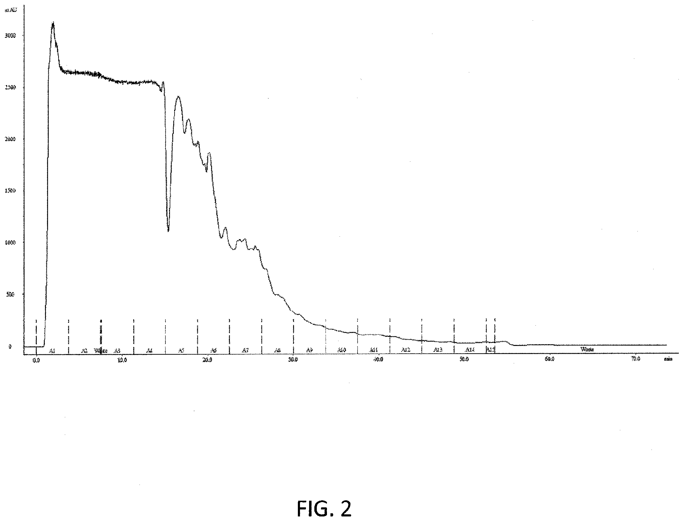

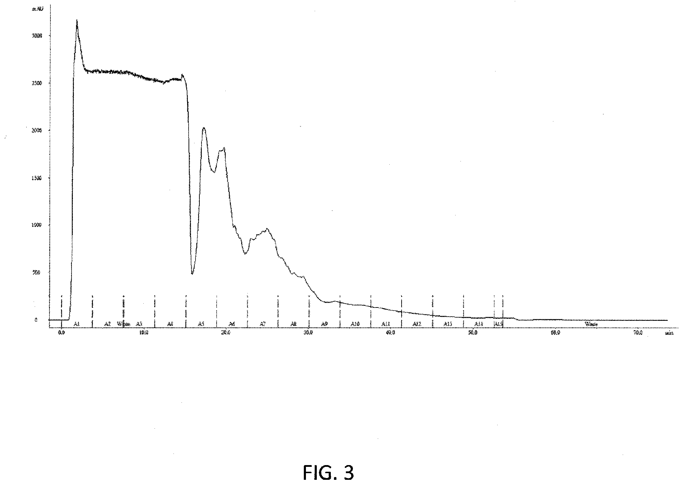

With reference to FIGs. 2 and 3, resulting SCX UV traces with the UV recorded at 214 nm are shown for a Saliva sample and GCF sample respectively. The Saliva and GCF samples were then desalted with the Macrotrap LC column as above, vacuum centrifuged and re-suspended in 200µL of 0.1% formic acid. 20µL of the samples were desalted with two ziptips and eluted in 20µL.

### Fraction Analysis

Each fraction was analysed in triplicate by LC-MS/MS. Peptides were loaded onto a 150mm Acclaim PepMap100 C18 column in mobile phase A (0.1% formic acid). Peptides were separated over a linear gradient from 3.2% to 44% mobile phase B (acetonitrile + 0.1% formic acid) with a flow rate of 350nl/min. The column was then washed with 90% mobile phase B before re-equilibrating at 3.2% mobile phase B. The column oven was heated to 35°C. The LC system was coupled to an Advion Triversa Nanomate (Advion, Ithaca, NY) which infused the peptides with a spray voltage of 1.7 kV. Peptides were infused directly into the LTQ-Orbitrap Velos ETD (Thermo Fischer Scientific, Bremen, Germany). The mass spectrometer performed a full FT-MS scan (*m*/*z* 380-1600) and subsequent collision induced dissociation (CID) MS/MS scans of the three most abundant ions followed by higher energy collisional dissociation (HCD) of the same three ions. The CID spectra were used to identify the peptides and the HCD spectra were used to quantify them.

### Data analysis

The data were analyzed using Proteome Discoverer (V1.2, Thermo Fisher Scientific). Data were analyzed as the technical repeats. The Mascot and SEQUEST algorithms were used to search the data with identical setting used. The database was the IPI human database supplemented with oral bacteria as described by Socransky. This database was concatenated with a reverse version to provide false discovery rates. The data were searched with the following settings: semi-trypsin was selected as the enzyme with a maximum of 2 missed cleavages, 5 ppm mass accuracy for the precursor ion, fragment ion mass tolerance was set to 0.5 Da. Carboxyamidomethylation of cysteine and iTRAQ addition to the N-terminus and lysine residues were set as a static modification. Phosphorylation of serine, threonine and tyrosine was set as a variable modification as was oxidation of methionine and iTRAQ addition to tyrosine.

The search results from each of the technical replicates were combined and proteins which were identified with two or more peptides were classed as identified. Only unique peptides were used for protein quantification and protein grouping was employed (only proteins which contained unique peptides were used).

### GCF ANALYSIS PRELIMINARY RESULTS - DISCOVERED PROTEINS

From the analysis of all GCF samples, 270 proteins were identified with two or more peptides. This included 264 human proteins and 6 bacterial proteins. The identified proteins are shown along with relative quantification values in the Appendix, Supplemental Table 1. All proteins show ratios relative to the Healthy control group (label 113- health). This data was subsequently normalized to collected GCF volumes and also log transformed (base 2) to give positive and negative abundance values.

There were no proteins which were solely identified in any of the disease states. The majority of the proteins showed a decrease in abundance between health, gingivitis and disease (229 proteins were lower abundance in gingivitis compared to health, 195 in mild periodontitis and 174 in severe periodontitis). This decrease in abundance across the groups may be due to an increase in GCF volume as tissues become more inflamed and as evidenced in Table 2. Alternatively, a "non-normalized" analysis of GCF may be performed to address this issue, which is recognized in the literature (Lamster *et al* 1986, Chapple *et al* 1994 & 1999).

### GCF CLUSTERING ANALYSIS PERFORMED ON DISCOVERED PROTEINS

Discovered proteins were clustered using the PolySNAP 3 software. PolySNAP 3 compares each 1 dimensional protein profile with every other and uses a weighted mean of Pearson parametric and Spearman nonparametric correlation coefficients to produce similarity scores. The profiles were clustered using a combination of statistical methods including principle component analysis, gamma statistics, and metric multidimensional scaling (MMDS). The data were then visualized in dendrograms, PCA plots, and MMDS plots. In this analysis, the group average link hierarchical clustering and complete link hierarchical clustering methods were used to group the data. In all cases, the number of clusters used was automatically set by PolySNAP3.

From the group average clustering three rounds of clustering were performed. The group with the largest number of proteins was re-clustered at each point.

### First Round of Clustering

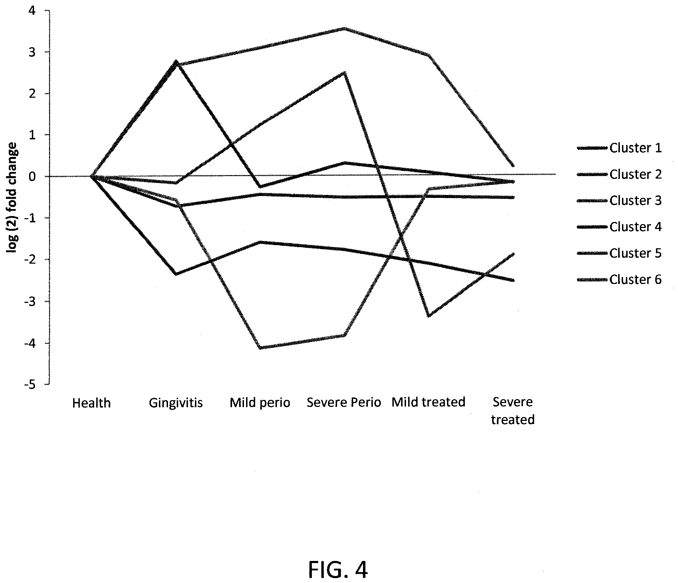

With reference to FIG. 4, the first round of analysis provided 6 clusters. Cluster 1 contained the majority of the proteins (243 proteins), cluster 2 contained 19 proteins, clusters 3, 5 and 6 each contained only one protein and cluster 4 contained five proteins.

With continuing reference to FIG. 4, Cluster 4 may be of interest as it includes a set of proteins which decrease in abundance during disease but do not return to baseline post-resolution. The nineteen proteins identified as cluster 2 show an increase in abundance with gingivitis before returning to baseline like levels in periodontitis. This may be due to one of the GCF samples containing blood, however bleeding is a critical clinical sign of gingivitis and periodontitis and blood-related proteins may be very discriminatory between health and disease. There are several blood related proteins identified in this group including haemoglobin alpha and beta. This could be of interest as a group that could distinguish between gingivitis and periodontitis and health, notwithstanding the possible presence of blood. Clusters 3 and 5 for example appear to distinguish untreated periodontitis from health/gingivitis.

The proteins identified clusters of interest, clusters 3, 4, and 5, are shown in the Appendix, Supplementary Table 2.

### Second Round of Clustering

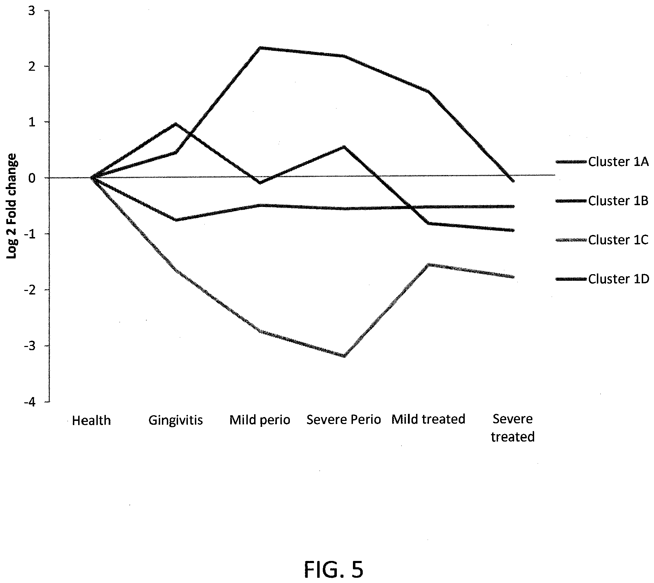

With reference to FIG. 5, the 243 proteins from cluster 1 of FIG. 4 were re-clustered, which gave a total of four groups. Group 1A contained the majority of the proteins (233), groups 1B and 1C contained two proteins each and group ID contained six proteins. Group ID shows little change between health and gingivitis before increasing with periodontitis. There is a fall in relative abundance between mild periodontitis and treated mild periodontitis and a return to baseline in the treated severe periodontitis. The two proteins identified in group 1C appear to follow disease, with a decrease to gingivitis and a larger decrease to the two perio groups before returning towards the baseline in the treated samples. Such proteins could be envisaged as being analyzed as outcome measures of whether treatment was successful or not.

The proteins identified in clusters of interest, clusters 1C and 1D, are shown in the Appendix, Supplementary Table 3.

### Third Round of Clustering

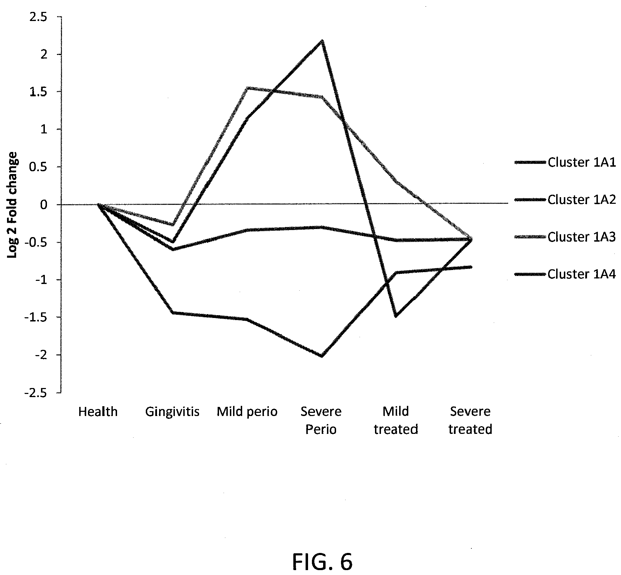

With reference to FIG. 6, the 233 proteins from cluster 1A of FIG. 5 were clustered again, resulting in four clusters, though the change in abundance is now less than 2 on the log scale (4 times increase/decrease). The largest cluster (1A1) still contained 171 proteins, cluster 1A2 contained 50 proteins, 1A3 contained 10 and 1A4 contained 2 proteins. Again there appear to be groups of potential interest in this analysis.

The proteins identified in each cluster of interest, 1A3 and 1A4, are shown in the Appendix, Supplementary Table 4.

### Final Round of Clustering

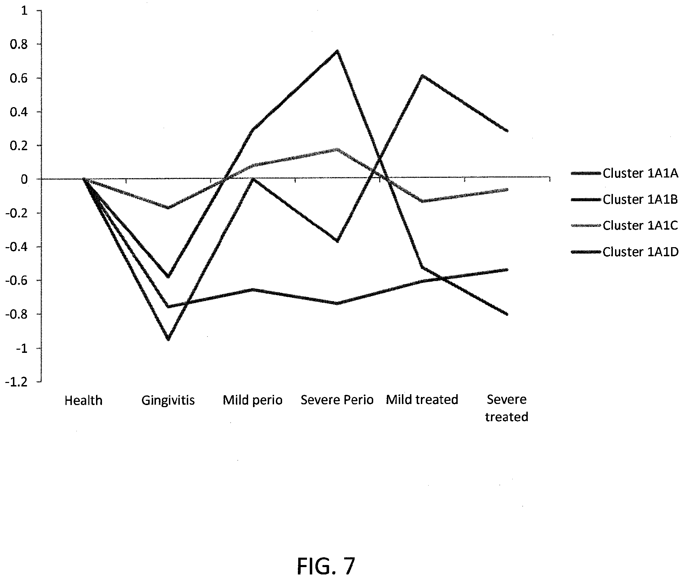

With reference to FIG. 7, a final round of clustering was performed the 171 proteins from cluster 1A1 of FIG. 6. This resulted in 4 groups as shown in Figure 6. There are no clusters from this analysis which appear of interest.

The multiple rounds of clustering analysis suggest that there are some groups of proteins in GCF which may distinguish between different disease states of periodontitis.

### SALIVA ANALYSIS PRELIMINARY RESULTS - DISCOVERED PROTEINS

All saliva samples were analyzed similarly to GCF samples. 314 proteins were identified with two or more peptides, including 307 human proteins and 7 bacterial proteins. One protein was identified in only one sample group (edentulous). The identified proteins are shown along with relative quantification values in the Appendix, Supplemental Table 5.

### SALIVA CLUSTERING ANALYSIS PERFORMED ON DISCOVERED PROTEINS

### First Round of Clustering

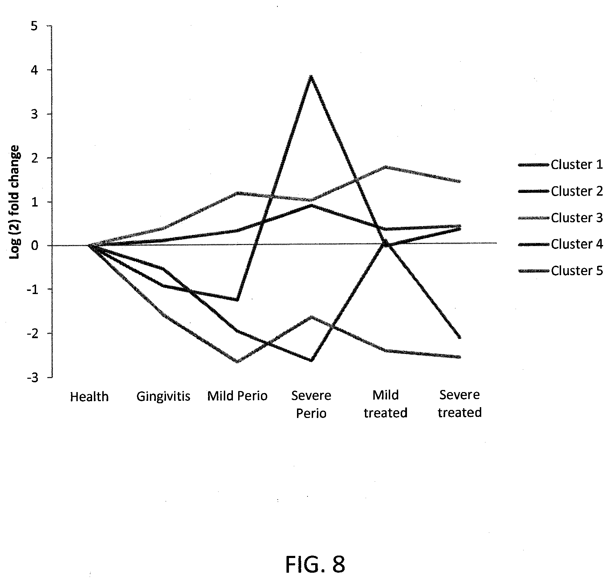

For the clustering analysis the edentulous samples were not included. Clustering analysis was performed using PolySNAP3. With reference to FIG. 8, the first round of clustering resulted in five clusters. The largest cluster (1) contained 297 proteins, while clusters 2 and 5 each contained one protein. Cluster 3 contained 11 proteins and cluster 4 contained three proteins. As with the GCF dataset there is a group of proteins which are down-regulated with disease and do not return to baseline following treatment. Cluster 2 appears to distinguish severe periodontitis from milder conditions.

The proteins identified in the cluster of interest, cluster 2, is shown in the Appendix, Supplementary Table 6.

### Sound Round of Clustering

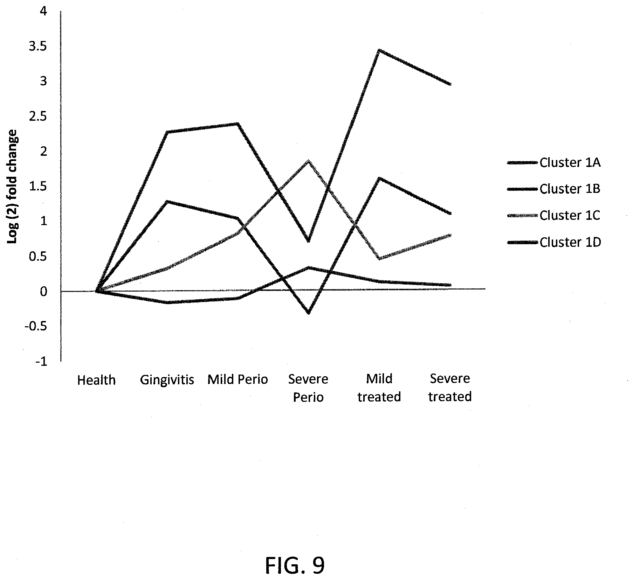

With reference to FIG. 9, Cluster 1 from FIG. 8 was re-clustered resulting in an additional 4 groups. The largest group contained 166 proteins (cluster 1A). Clusters IB and ID contained 14 and one protein respectively. These two groups may distinguish between gingivitis/mild periodontitis and severe periodontitis. However, in both cases, the signal for severe periodontitis is close to healthy levels, though after treatment an increase in protein abundance for both mild and severe periodontitis occurs. Cluster 1C contained 116 proteins. In this group, little change is shown between health and gingivitis followed by an increase to mild periodontitis before a large increase to severe periodontitis. These values are reduced in the treated samples but still at greater levels than the gingivitis group.

The proteins identified in each cluster of interest, clusters 1B and 1D, are shown in the Appendix, Supplementary Table 7.

### Third Round of Clustering

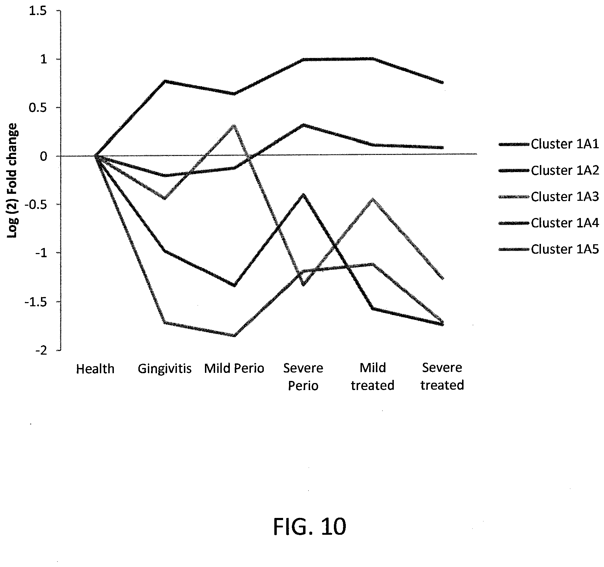

With reference to FIG. 10, cluster 1A from FIG. 9 was re-clustered. Cluster 1A gave resulted in 5 groups, the largest cluster (1A1) containing 150 proteins. There do not appear to be any significant clusters here. Cluster 1A4 provided 3 proteins, and Cluster 1A5 provided one protein. The protein biomarkers in Clusters 1A4 and 1A5 all show an increase or decrease in protein abundance between health and gingivitis which is greater in mild periodontitis but less in severe periodontitis.

The proteins identified in clusters 1A4 and 1A5 are shown in the Appendix, Supplementary Table 8.

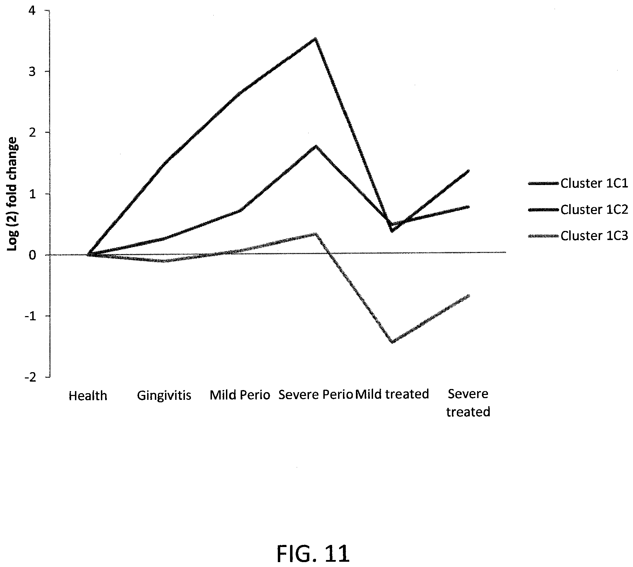

With reference to FIG. 11, cluster 1C from FIG. 9 was re-clustered, resulting in three clusters. Cluster 1C2 contained 7 proteins showing a near linear increase in proteins abundance up to severe periodontitis before a reduction post treatment. This group was not clustered any further. The proteins identified in cluster 1C2 are shown in the Appendix, Supplementary Table 9.

### Final Round of Clustering

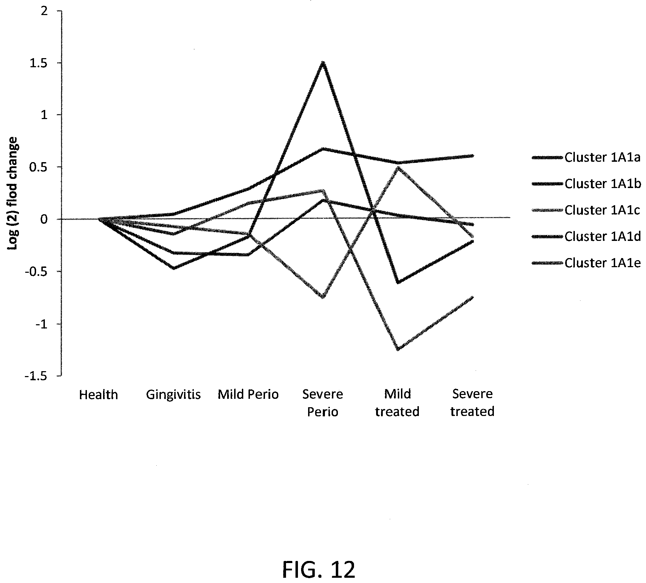

With reference to FIG. 12, cluster 1A1 from FIG. 10 was re-clustered. Five clusters were observed with a group of proteins showing an increase in abundance for severe periodontitis (cluster 1A1b) but none of the other groups. There were five proteins identified in this cluster. The proteins identified in cluster 1a1b are shown in the Appendix, Supplementary Table 10.

### COMPARISON OF GCF AND SALIVA DATASETS

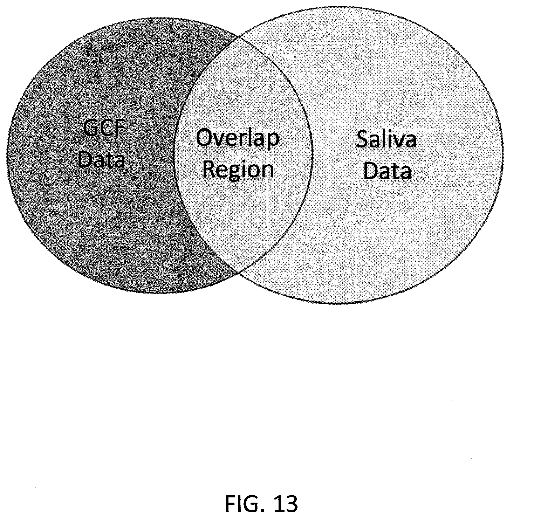

The proteins observed in the two data sets were compared to identify protein biomarkers that were discovered in both saliva and GCF samples. With reference to FIG. 13, 95 proteins were identified in both the GCF and saliva, represented by the overlapping region of the Venn diagram of FIG. 13. This is approximately a third of the total number of proteins identified on the GCF dataset.

The proteins which are observed in the overlapping region are shown in the Appendix, Supplemental Table 11. The associated abundance data for the proteins in Supplemental Table 11 was collected and subsequently transformed to portray the log (2) ratios for the protein abundance observed. Additionally, two values for the GCF was measured, one normalized to the volume collected, and the other not.

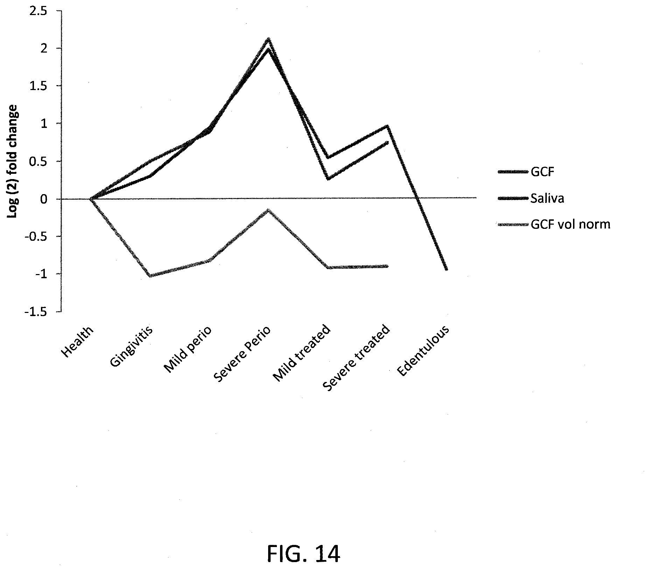

If it is assumed that GCF is a component of saliva, and when saliva is not normalized to the same GCF volumes, it may be of use to compare the three values. Analysis of these triple values shows some of these proteins to have very similar profile. Some of those protein biomarkers with a large increase or decrease in abundance values are depicted in FIGS. 14-17.

FIG. 14 shows the three traces for protein S100-P. S100-P is involved in the regulation of cell cycle progression and differentiation. It has been observed in both GCF and saliva and has been suggested as a potential biomarker for oral squamous cell carcinoma. As shown in FIG. 14, iTRAQ measured abundance of S100-P protein show there is a large increase between mild periodontitis and severe periodontitis. Accordingly, S100-P may serve as a useful protein biomarker for distinguishing between mild and severe periodontitis.

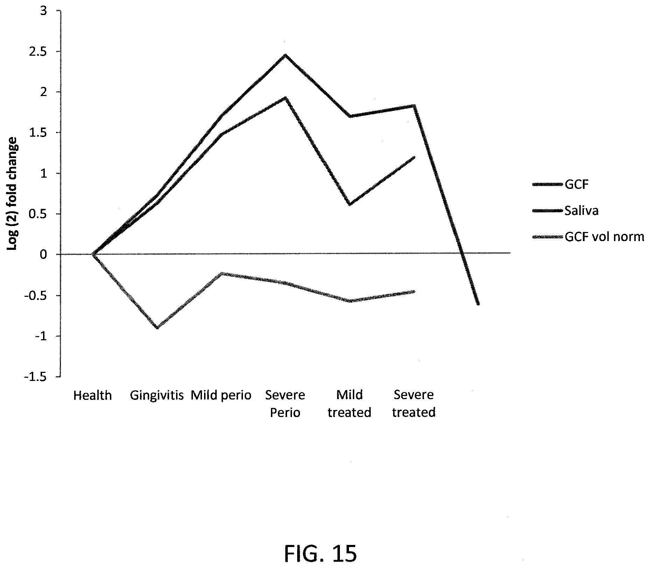

FIG. 15 shows the three traces for protein S100-A8, which is also known as calgranulin-A. It has antimicrobial activity towards bacteria. It is a pro-inflammatory mediator in inflammation and up-regulates the release of IL8. High levels of S100-A8 have been detected in the plasma of patients with chronic periodontitis. As shown in FIG. 15, iTRAQ measured abundanceof S100-A8 protein show there is a large increase between mild periodontitis and severe periodontitis. Accordingly, S100-A8 may serve as a useful protein biomarker for distinguishing between mild and severe periodontitis.

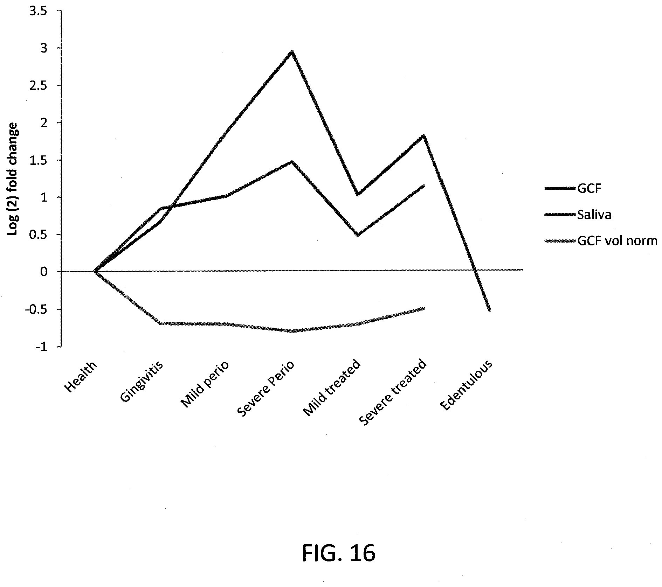

FIG. 16 shows the three traces for protein myosin-9. As shown in FIG. 16, iTRAQ measured abundance of myosin-9 protein show there is a large increase between mild periodontitis and severe periodontitis. Accordingly, myosin-9 may serve as a useful protein biomarker for distinguishing between mild and severe periodontitis.

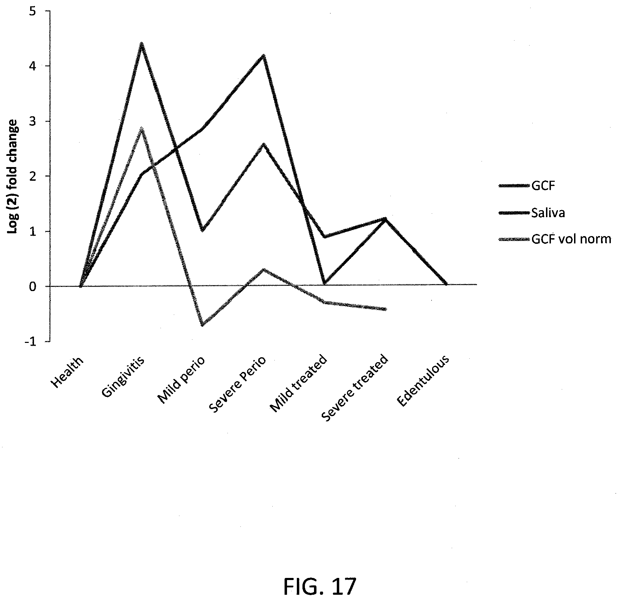

FIG. 17 shows the three traces for protein transaldolase. As shown in FIG. 17, iTRAQ measured abundance of transaldolase protein show there is a large increase between mild periodontitis and severe periodontitis. Accordingly, transaldolase may serve as a useful protein biomarker for distinguishing between mild and severe periodontitis.

FIG. 18 shows the three traces for protein haemoglobin beta. As shown in FIG. 18, iTRAQ measured abundance of haemoglobin beta protein show there is a large increase between mild periodontitis and severe periodontitis. The traces also increase and decrease throughout the range of all oral disease states. Accordingly, haemoglobin beta (or alpha) may serve as a useful protein biomarker for distinguishing between mild and severe periodontitis, and/or other oral disease states.

### DISCUSSION OF RESULTS

Gene ontology analysis using The Database for Annotation, Visualization and Integrated Discovery (DAVID) on the GCF and Saliva datasets shows that the most significantly enriched biological process in the saliva dataset were the defense responses, and in GCF dataset, was cytoskeletal organization. The top twenty processes are shown in the Appendix, Supplemental Table 12. Seven of the twenty are enriched in both GCF and saliva including defense responses, responses to stimuli, and glycolysis.

The analysis of GCF and saliva identified 270 proteins in GCF and 314 proteins in saliva of which 95 were identified in both. All proteins except one (solely identified in edentulous saliva) were quantified over the different disease and resolution phases. Of the proteins which are identified in both GCF and saliva there are several proteins which show increases (in both GCF and Saliva datasets) with disease which could potentially be used to distinguish between health, gingivitis, mild and severe periodontitis and resolution of disease.

According to an exemplary embodiment, a method for diagnosing a status of an oral disease includes selecting at least one protein biomarker from the group consisting of: haemoglobin chains alpha and beta, carbonic anhydrase 1(IPI00980674), and plastin-1. The method may further include diagnosing the status at least one of a healthy state, gingivitis state, and a mild and/or severe periodontitis state. In another embodiment, the at least one protein biomarker is selected from the group consisting of the protein biomarkers in saliva data cluster 1C2 (Supplemental Table 9): Protein #IPI00016347.5, Protein #IPI00377122.4, haemoglobin subunit alpha (IPI00410714.5), haemoglobin subunit delta (IPI00473011.3), haemoglobin subunit beta (IPI00654755.3), protein # IPI00980674.1, and protein accession number #O83773.

There are also several protein biomarkers which are potential indicators for severe periodontitis by showing increases in abundance in both the GCF and saliva datasets. In an exemplary embodiment, a method for diagnosing severe periodontitis includes selecting at least one protein biomarker from the group consisting of: S100-P, transaldolase, S100-A8 (calgranulin-A), myosin-9, haemoglobin alpha, and haemoglobin beta. In another aspect, the method for diagnosing severe periodontitis includes selecting at least one protein biomarker from the group consisting of the protein biomarkers in saliva data cluster 1a1b (Supplemental Table 10): Protein S100-A11 (IPI00013895.1), Protein IPI00037070.3, catalase (IPI00465436.4), Choline transporter-like protein 2 derivative (IPI00903245.1), and titin isoform N2-B (IPI00985334.2).

In yet another aspect, the method for diagnosing severe periodontitis includes selecting at least one protein biomarker from the group consisting of: S100-P, transaldolase, S100-A8 (calgranulin-A), myosin-9, haemoglobin alpha, and haemoglobin beta, alpha-1-acid glycoprotein 1 and 2, matrix metalloproteinase-9, peptidyl-prolyl cis-trans isomerase A and haptoglobin-related protein (IPI00431645.1).

According to another exemplary embodiment, a method for diagnosing gingivitis or mild periodontitis includes selecting at least one protein biomarker from the group consisting of the protein biomarkers in saliva data clusters 1B, 1D (Supplementary Table 7) and/or in saliva data clusters 1A4, 1A5 (Supplementary Table 8). These protein biomarkers all show an increase or decrease in protein abundance between health and gingivitis which is greater in mild periodontitis but less in severe periodontitis. It may be possible to use these to differentiate between gingivitis and mild periodontitis with severe periodontitis.

According to another aspect, a method for diagnosing gingivitis or mild periodontitis includes selecting at least one protein biomarker from NADPH oxidase activator-1 and alpha-N-acetylgalactosaminidase. NADPH oxidase activator-1 is involved in the production of reactive oxygen species. alpha-N-acetylgalactosaminidase portrays has some of the highest ratios for gingivitis and mild periodontitis compared to severe periodontitis. This protein is involved in the breakdown of glycolipids. In another aspect, the method for diagnosing gingivitis or mild periodontitis includes selecting an Alphaa alpha-N-acetylgalactosaminidase biomarker.

### Clauses

1. A method for diagnosing the status of periodontitis disease, comprising:
   providing at least one of a gingival crevicular fluid (GCF) sample and a saliva sample;
   selecting a set of protein biomarkers for identifying a particular state of periodontitis; and
   determining the expression levels in the selected set of protein biomarkers to diagnose the status of periodontitis disease.
2. The method according to claim 1, wherein the set of protein biomarkers is selected for distinguishing between a gingivitis state and a periodontitis state.
3. The method according to claim 1, wherein the set of protein biomarkers is selected for distinguishing between a periodontal health and a disease state.
4. The method according to claim 1, wherein the set of protein biomarkers is selected for distinguishing between a mild periodontitis state and a severe periodontitis state.
5. The method according to any one of claims 1-4, wherein the set of protein biomarkers includes at least one protein selected from the group consisting of haemoglobin chains alpha and beta, carbonic anhydrase 1(International Protein Index or "IPI" #IPI00980674), and plastin-1.
6. The method according to any one of claims 1-4, wherein the set of protein biomarkers includes at least one protein selected from the group consisting of S100-P, transaldolase, S100-A8 (calgranulin-A), myosin-9, Haemoglobin Alpha, and Haemoglobin Beta.
7. The method according to any one of claims 1-4, wherein the set of protein biomarkers includes at least one protein selected from the group consisting of Alpha-1-acid glycoprotein 1 and 2, matrix metalloproteinase-9, Peptidyl-prolyl cis-trans isomerase A, and Haptoglobin-related protein (IPI00431645.1).
8. The method according to any one of claims 1-4, wherein the set of protein biomarkers includes at least one protein selected from the group consisting of NADPH oxidase and Alpha-N-acetylgalactosaminidase.
9. The method according to any one of claims 1-4, wherein the set of protein biomarkers includes Alpha-N-acetylgalactosaminidase.
10. The method according to any one of claims 1-4, wherein the set of protein biomarkers includes at least one protein selected from the group consisting of Protein S100-A11 (IPI00013895.1), Protein IPI00037070.3, catalase (IPI00465436.4), Choline transporter-like protein 2 derivative (IPI00903245.1), and titin isoform N2-B (IPI00985334.2).
11. The method according to any one of claims 5-8 and 10, wherein the set of protein biomarkers includes two or more biomarkers.
12. A kit for diagnosing the status of periodontitis disease, comprising a set of protein biomarkers selected to distinguish between gingivitis and periodontitis.
13. The kit according to claim 12, wherein the set of protein biomarkers includes at least one protein selected from the group consisting of haemoglobin chains alpha and beta, carbonic anhydrase 1(International Protein Index or "IPI" #IPI00980674), and plastin 1.
14. The kit according to claim 12, wherein the kit diagnoses gingivitis or mild periodontitis, and the set of protein biomarkers further includes at least one protein biomarkers from saliva data clusters 1B, ID, 1A4, and 1A5.
15. The method according to any one of claims 1-11, further including, further including:
   providing both the GCF sample and saliva sample;
   generating a first and second protein profile by analyzing the proteome of a GCF sample and a saliva sample;
   determining an overlap region between the first and second protein profiles;
   wherein selecting the set of protein biomarkers for distinguishing between particular states of periodontitis includes calculating a change in abundance of proteins within the overlap region during different stages of periodontitis and selecting those proteins which are under or over expressed during a single state of periodontitis.
16. The method according to any one of claims 1-11, further including:
   generating a protein profile by analyzing the proteome of the at least one oral fluid sample; and
   clustering the protein profile to determine a set of protein biomarkers.

### APPENDIX

**SUPPLEMENTARY TABLE 1**

| Accession | Description | A6: 114/113 | A6: 115/113 | A6: 116/113 | A6: 117/113 | A6: 118/113 |
|---|---|---|---|---|---|---|
| A8AW99 | Phosphoenolpyruvate carboxylase OS=Streptococcus gordonii (strain Challis / ATCC 35105 / CH1 / DL1 / V288) GN=ppc PE=3 SV=1 | 1.091 | 2.214 | 2.994 | 1.390 | 1.661 |
| A7I0P7 | Peptide chain release factor 2 OS=Campylobacter hominis (strain ATCC BAA-381/ LMG 19568 / NCTC 13146 / CH001A) GN=prfB PE=3 SV=1 - [RF2_CAMHC] | 2.185 | 6.140 | 9.502 | 1.589 | 2.120 |
| A8AX28 | tRNA pseudouridine synthase B OS=Streptococcus gordonii (strain Challis / ATCC 35105 / CH1 / DL1 / V288) GN=truB PE=3 SV=1 - [TRUB_STRGC] | 2.447 | 7.298 | 12.453 | 2.095 | 1.862 |
| A8AW24 | Isoleucine--tRNA ligase OS=Streptococcus gordonii (strain Challis / ATCC 35105 / CH1 / DL1 / V288) GN=ileS PE=3 SV=1 | 1.378 | 2.731 | 3.417 | 0.891 | 0.519 |
| IPI00003269.1 | Beta-actin-like protein 2 | 1.174 | 2.429 | 4.466 | 0.977 | 1.590 |
| IPI00003935.6 | Histone H2B type 2-E | 1.704 | 3.139 | 7.450 | 1.589 | 1.710 |
| IPI00004550.5 | Keratin, type I cytoskeletal 24 | 0.868 | 2.271 | 3.037 | 0.915 | 1.134 |
| IPI00005721.1 | Neutrophil defensin 1 | 1.062 | 0.949 | 1.070 | 0.923 | 1.599 |
| IPI00006988.1 | Resistin | 2.419 | 0.897 | 0.642 | 0.961 | 2.719 |
| IPI00007047.1 | Protein S100-A8 | 1.545 | 2.762 | 3.767 | 1.515 | 2.262 |
| IPI00008359.2 | Keratin, type II cytoskeletal 2 oral | 0.940 | 1.217 | 1.617 | 1.403 | 1.828 |
| IPI00008405.5 | Arylsulfatase F | 1.650 | 3.529 | 5.536 | 1.426 | 2.000 |
| IPI00008895.1 | Epithelial membrane protein 2 | 6.951 | 13.266 | 2.545 | 10.122 | 6.138 |
| IPI00009724.3 | Isoform 1 of EF-hand calcium-binding domain-containing protein 6 | 1.471 | 8.313 | 13.940 | 1.860 | 1.369 |
| IPI00009865.4 | Keratin, type I cytoskeletal 10 | 0.935 | 1.487 | 1.166 | 1.344 | 1.038 |
| IPI00009866.7 | Isoform 1 of Keratin, type I cytoskeletal 13 | 0.918 | 1.528 | 1.612 | 1.242 | 1.393 |
| IPI00009867.3 | Keratin, type II cytoskeletal 5 | 1.269 | 2.339 | 2.862 | 1.999 | 2.119 |
| IPI00010133.3 | Coronin-1A | 2.069 | 2.459 | 4.193 | 1.629 | 2.510 |
| IPI00010349.1 | Alkyldihydroxyacetonephosphate synthase, peroxisomal | 2.729 | 12.079 | 12.838 | 11.323 | 1.586 |
| IPI00010471.6 | Plastin-2 | 1.663 | 3.060 | 5.513 | 1.635 | 2.622 |
| IPI00013163.1 | Myeloid cell nuclear differentiation antigen | 2.025 | 2.914 | 5.563 | 1.109 | 1.678 |
| IPI00013890.2 | Isoform 1 of 14-3-3 protein sigma | 1.877 | 4.014 | 7.830 | 1.566 | 1.574 |
| IPI00013895.1 | Protein S100-A11 | 1.011 | 0.550 | 0.480 | 1.003 | 2.233 |
| IPI00017526.1 | Protein S100-P | 1.411 | 1.835 | 4.329 | 1.192 | 1.657 |
| IPI00019038.1 | Lysozyme C | 1.427 | 2.107 | 2.605 | 1.293 | 1.787 |
| IPI00019359.4 | Keratin, type I cytoskeletal 9 | 1.547 | 1.827 | 1.793 | 1.385 | 1.341 |
| IPI00019502.3 | Isoform 1 of Myosin-9 | 1.787 | 2.006 | 2.761 | 1.386 | 2.194 |
| IPI00019580.1 | Plasminogen | 2.288 | 1.643 | 1.937 | 1.261 | 1.948 |
| IPI00019869.3 | Protein S100-A2 | 8.362 | 3.474 | 4.944 | 2.218 | 2.994 |
| IPI00020091.1 | Alpha-1-acid glycoprotein 2 | 1.461 | 0.453 | 0.519 | 0.699 | 1.969 |
| IPI00021263.3 | 14-3-3 protein zeta/delta | 1.935 | 2.378 | 4.007 | 1.337 | 2.087 |
| IPI00021439.1 | Actin, cytoplasmic 1 | 1.532 | 3.145 | 4.896 | 1.445 | 2.320 |
| IPI00021828.1 | Cystatin-B | 1.026 | 1.725 | 1.936 | 1.094 | 1.383 |
| IPI00021841.1 | Apolipoprotein A-I | 2.054 | 1.828 | 3.046 | 1.710 | 2.498 |
| IPI00022371.1 | Histidine-rich glycoprotein | 2.157 | 1.828 | 2.648 | 1.880 | 2.269 |
| IPI00022429.3 | Alpha-1-acid glycoprotein 1 | 1.524 | 0.515 | 0.601 | 0.977 | 2.217 |
| IPI00022463.2 | Serotransferrin | 2.035 | 1.860 | 2.638 | 1.763 | 2.374 |
| IPI00022488.1 | Hemopexin | 2.864 | 2.697 | 3.481 | 1.933 | 3.455 |
| IPI00022974.1 | Prolactin-inducible protein | 1.786 | 1.298 | 1.464 | 1.495 | 3.193 |
| IPI00025512.2 | Heat shock protein beta-1 | 1.152 | 1.258 | 1.517 | 1.471 | 2.055 |
| IPI00027462.1 | Protein S100-A9 | 1.380 | 2.491 | 2.812 | 1.513 | 2.136 |
| IPI00027463.1 | Protein S100-A6 | 2.846 | 4.107 | 3.748 | 2.172 | 4.088 |
| IPI00027509.5 | Matrix metalloproteinase-9 | 2.216 | 3.561 | 3.127 | 2.021 | 1.365 |
| IPI00027769.1 | Neutrophil elastase | 1.836 | 2.667 | 9.024 | 1.281 | 1.919 |
| IPI00028064.1 | Cathepsin G | 1.391 | 1.130 | 1.206 | 1.072 | 1.571 |
| IPI00030362.1 | Isoform 1 of Proteolipid protein 2 | 1.389 | 2.013 | 4.137 | 1.411 | 1.791 |
| IPI00032294.1 | Cystatin-S | 0.657 | 0.573 | 0.729 | 0.853 | 1.867 |
| IPI00037070.3 | Uncharacterized protein | 2.165 | 0.946 | 1.272 | 1.135 | 1.887 |
| IPI00081836.3 | Histone H2A type 1-H | 1.107 | 0.716 | 1.909 | 1.903 | 3.763 |
| IPI00152758.1 | FLJ00198 protein (Fragment) | 23.303 | 4.014 | 7.989 | 2.169 | 2.697 |
| IPI00167191.1 | CDNA FLJ25707 fis, clone TST04879 | 1.935 | 0.186 | 0.337 | 1.789 | 2.778 |
| IPI00168728.1 | FLJ00385 protein (Fragment) | 2.284 | 9.149 | 13.295 | 3.104 | 2.935 |
| IPI00174541.1 | Isoform 4 of Interleukin-1 receptor antagonist protein | 1.402 | 0.948 | 1.229 | 1.205 | 2.084 |
| IPI00177428.1 | Isoform 2 of Mitochondrial intermembrane space import and assembly protein 40 | 1.091 | 0.307 | 0.335 | 0.775 | 0.854 |
| IPI00180240.2 | Thymosin beta-4-like protein 3 | 1.819 | 2.538 | 4.657 | 1.818 | 2.105 |
| IPI00216691.5 | Profilin-1 | 2.126 | 3.193 | 4.898 | 1.993 | 3.376 |
| IPI00216974.1 | Isoform 1 of Probable phospholipid-transporting ATPase IK | 37.175 | 0.920 | 3.993 | 1.491 | 1.460 |
| IPI00217465.5 | Histone H1.2 | 3.025 | 3.780 | 6.682 | 2.143 | 3.026 |
| IPI00217468.3 | Histone H1.5 | 1.816 | 3.043 | 4.613 | 1.645 | 2.749 |
| IPI00217963.3 | Keratin, type I cytoskeletal 16 | 0.906 | 1.190 | 1.304 | 1.299 | 1.537 |
| IPI00218131.3 | Protein S100-A12 | 1.831 | 2.688 | 8.152 | 1.919 | 2.218 |
| IPI00218918.5 | Annexin A1 | 1.310 | 2.384 | 2.914 | 1.317 | 1.806 |
| IPI00219037.5 | Histone H2A.x | 2.459 | 3.327 | 8.477 | 1.536 | 2.063 |
| IPI00219208.1 | Isoform 2 of Granulocyte-macrophage colony-stimulating factor receptor subunit alpha | 1.797 | 2.298 | 4.347 | 1.840 | 2.175 |
| IPI00219395.3 | Isoform 6 of Voltage-dependent T-type calcium channel subunit alpha-1G | 3.160 | 6.602 | 10.369 | 2.696 | 4.044 |
| IPI00219502.1 | Isoform Short of Gl/S-specific cyclin-E2 | 1.657 | 1.027 | 1.319 | 1.477 | 4.004 |
| IPI00219757.13 | Glutathione S-transferase P | 1.563 | 2.730 | 4.126 | 1.820 | 2.059 |
| IPI00220056.1 | Kelch domain-containing protein 5 | 1.232 | 1.456 | 2.136 | 1.420 | 1.499 |
| IPI00220327.4 | Keratin, type II cytoskeletal 1 | 1.116 | 1.181 | 1.449 | 1.163 | 1.170 |
| IPI00236554.1 | Isoform H14 of Myeloperoxidase | 2.434 | 4.087 | 10.319 | 1.735 | 2.189 |
| IPI00290077.3 | Keratin, type I cytoskeletal 15 | 0.981 | 0.876 | 0.777 | 1.045 | 1.535 |
| IPI00292579.4 | Stabilin-2 | 5.188 | 18.978 | 17.247 | 7.542 | 2.843 |
| IPI00293665.9 | Keratin, type II cytoskeletal 6B | 0.924 | 1.657 | 1.791 | 1.741 | 1.746 |
| IPI00296215.2 | Epithelial cell adhesion molecule | 13.055 | 0.830 | 1.428 | 1.586 | 1.096 |
| IPI00297056.2 | Cornulin | 1.613 | 2.387 | 2.849 | 1.361 | 1.963 |
| IPI00297763.4 | Retinal-specific ATP-binding cassette transporter | 1.662 | 3.618 | 7.512 | 1.030 | 1.452 |
| IPI00298497.3 | Fibrinogen beta chain | 1.627 | 1.774 | 2.386 | 1.309 | 1.816 |
| IPI00299078.1 | Salivary acidic proline-rich phosphoprotein 1/2 | 0.684 | 0.967 | 0.989 | 1.197 | 1.396 |
| IPI00299547.4 | Isoform 1 of Neutrophil gelatinase-associated lipocalin | 2.854 | 3.215 | 8.337 | 1.534 | 3.224 |
| IPI00300725.7 | Keratin, type II cytoskeletal 6A | 0.909 | 1.345 | 1.079 | 2.018 | 1.701 |
| IPI00305477.6 | Cystatin-SN | 1.286 | 1.089 | 1.617 | 1.253 | 2.407 |
| IPI00307770.2 | Uncharacterized protein | 1.106 | 1.956 | 1.584 | 1.259 | 1.019 |
| IPI00328296.2 | PDZ domain-containing protein GIPC2 | 0.765 | 0.759 | 0.811 | 0.735 | 0.923 |
| IPI00334400.2 | Isoform 2 of Plakophilin-4 | 0.374 | 0.797 | 1.320 | 0.616 | 0.541 |
| IPI00374975.2 | Probable phosphoglycerate mutase 4 | 0.857 | 0.479 | 0.362 | 1.082 | 2.494 |
| IPI00375293.2 | Isoform 2 of High affinity immunoglobulin gamma Fc receptor I | 2.130 | 7.149 | 20.796 | 0.789 | 2.361 |
| IPI00377081.1 | erythrocyte band 7 integral membrane protein isoform b | 1.951 | 2.869 | 5.214 | 1.399 | 1.858 |
| IPI00384282.2 | FERM, C-terminal PH-like domain containing protein | 1.804 | 2.321 | 1.684 | 1.562 | 2.468 |
| IPI00384444.6 | Keratin, type I cytoskeletal 14 | 1.098 | 1.767 | 1.755 | 1.580 | 1.937 |
| IPI00384938.1 | Putative uncharacterized protein DKFZp686N02209 | 3.048 | 3.013 | 4.415 | 2.739 | 3.220 |
| IPI00385373.1 | Truncated proliferating cell nuclear antigen | 2.277 | 5.425 | 10.797 | 1.879 | 3.952 |
| IPI00394951.1 | Putative ubiquitin carboxyl-terminal hydrolase 17-like protein 1 | 0.794 | 1.094 | 1.055 | 1.100 | 1.280 |
| IPI00397585.1 | Isoform 2 of Leucine-rich repeat LGI family member 4 | 1.485 | 5.536 | 11.795 | 1.242 | 1.470 |
| IPI00399007.7 | Putative uncharacterized protein DKFZp686I04196 (Fragment) | 2.176 | 4.694 | 8.528 | 2.583 | 4.705 |
| IPI00402502.2 | secernin-2 isoform 2 | 1.847 | 3.169 | 5.171 | 1.638 | 1.359 |
| IPI00410714.5 | Hemoglobin subunit alpha | 19.785 | 2.146 | 5.893 | 1.815 | 2.553 |
| IPI00418153.1 | Putative uncharacterized protein DKFZp686I15212 | 2.111 | 9.132 | 16.172 | 3.105 | 3.051 |
| IPI00418262.5 | Fructose-bisphosphate aldolase | 2.633 | 2.420 | 4.379 | 1.698 | 1.846 |
| IPI00419585.9 | Peptidyl-prolyl cis-trans isomerase A | 1.939 | 2.634 | 2.609 | 1.437 | 2.073 |
| IPI00423460.3 | Putative uncharacterized protein DKFZp686G21220 (Fragment) | 1.626 | 1.478 | 2.635 | 1.433 | 2.829 |
| IPI00431645.2 | 31 kDa protein | 1.488 | 2.257 | 3.870 | 1.899 | 3.274 |
| IPI00448925.6 | 44 kDa protein | 2.771 | 3.473 | 5.077 | 2.394 | 2.905 |
| IPI00449920.1 | cDNA FU90170 fis, clone MAMMA1000370, highly similar to Ig alpha-1 chain C region | 1.596 | 1.643 | 2.609 | 1.548 | 2.673 |
| IPI00450768.7 | Keratin, type I cytoskeletal 17 | 1.083 | 1.815 | 1.071 | 1.764 | 2.427 |
| IPI00453473.6 | Histone H4 | 1.803 | 2.130 | 5.598 | 1.308 | 1.478 |
| IPI00465248.5 | Isoform alpha-enolase of Alpha-enolase | 1.791 | 2.111 | 2.924 | 1.807 | 3.210 |
| IPI00465439.5 | Fructose-bisphosphate aldolase A | 1.640 | 3.506 | 5.165 | 1.472 | 2.197 |
| IPI00470476.3 | Uncharacterized protein C9orfl44A | 2.945 | 33.019 | 68.061 | 4.446 | 4.289 |
| IPI00477227.3 | Isoform 1 of Keratin, type II cytoskeletal 78 | 2.284 | 2.575 | 2.759 | 1.760 | 1.887 |
| IPI00477597.2 | Isoform 1 of Haptoglobin-related protein | 1.721 | 2.349 | 2.958 | 1.753 | 2.713 |
| IPI00478493.3 | haptoglobin isoform 2 preproprotein | 1.866 | 2.137 | 3.510 | 1.957 | 3.282 |
| IPI00478837.1 | Zinc finger protein 580 | 1.497 | 3.258 | 3.719 | 3.443 | 3.763 |
| IPI00479145.3 | Keratin, type I cytoskeletal 19 | 1.384 | 1.614 | 2.336 | 1.525 | 1.077 |
| IPI00479186.7 | Isoform M2 of Pyruvate kinase isozymes M1/M2 | 1.817 | 2.016 | 3.100 | 1.343 | 2.417 |
| IPI00513782.3 | cDNA FLJ35478 fis, clone SMINT2007796, highly similar to Gelsolin | 1.795 | 3.124 | 4.434 | 1.432 | 2.293 |
| IPI00514954.2 | Isoform 3 of Membrane-associated guanylate kinase, WW and PDZ domain-containing protein 3 | 19.533 | 2.306 | 5.063 | 1.736 | 2.091 |
| IPI00552280.2 | Bactericidal/permeability-increasing protein | 3.490 | 4.103 | 6.014 | 2.387 | 2.685 |
| IPI00552637.1 | G-protein coupled receptor | 3.109 | 9.833 | 29.016 | 1.776 | 3.499 |
| IPI00552768.1 | Uncharacterized protein | 2.467 | 3.130 | 4.076 | 2.048 | 2.971 |
| IPI00554648.3 | Keratin, type II cytoskeletal 8 | 0.951 | 1.135 | 1.151 | 1.521 | 1.629 |
| IPI00554711.3 | Junction plakoglobin | 2.401 | 1.725 | 1.711 | 1.645 | 1.444 |
| IPI00556287.1 | Putative uncharacterized protein | 3.141 | 9.484 | 8.840 | 4.033 | 1.990 |
| IPI00556432.1 | Oxysterol-binding protein (Fragment) | 1.367 | 2.816 | 2.088 | 2.288 | 2.156 |
| IPI00641244.1 | 11 kDa protein | 1.169 | 1.798 | 1.727 | 1.363 | 1.777 |
| IPI00641737.2 | Haptoglobin | 1.644 | 2.777 | 4.785 | 2.105 | 3.097 |
| IPI00642042.3 | Putative uncharacterized protein DKFZp686J1372 | 1.731 | 1.777 | 2.490 | 1.322 | 2.217 |
| IPI00642632.2 | Ig lambda-7 chain C region | 2.102 | 3.498 | 6.515 | 1.678 | 2.730 |
| IPI00642694.3 | cDNA FLJ55428, highly similar to Regulator of G-protein signaling 7 | 1.823 | 1.401 | 2.230 | 1.511 | 2.922 |
| IPI00644534.6 | Uncharacterized protein | 2.269 | 3.559 | 5.442 | 2.056 | 3.265 |
| IPI00645801.1 | Uncharacterized protein | 1.302 | 1.726 | 2.340 | 1.338 | 1.598 |
| IPI00654755.3 | Hemoglobin subunit beta | 21.011 | 1.997 | 5.888 | 1.833 | 2.307 |
| IPI00658130.1 | IGL@ protein | 2.182 | 3.474 | 6.670 | 1.802 | 2.970 |
| IPI00658186.1 | cDNA FLJ59463, highly similar to Geranylgeranyl pyrophosphate synthetase | 1.685 | 2.271 | 2.159 | 1.803 | 1.991 |
| IPI00739683.3 | Beta-defensin 131 | 0.976 | 3.084 | 7.562 | 1.011 | 0.950 |
| IPI00745280.1 | Similar to Keratin, type II cytoskeletal 7 | 4.024 | 8.529 | 10.940 | 3.927 | 5.645 |
| IPI00745872.2 | Isoform 1 of Serum albumin | 2.157 | 2.623 | 3.404 | 2.264 | 2.921 |
| IPI00746352.1 | SH3 domain-binding glutamic acid-rich-like protein 3 | 1.700 | 2.191 | 3.645 | 1.565 | 3.303 |
| IPI00748022.2 | Actin-like protein (Fragment) | 1.391 | 1.059 | 0.925 | 1.449 | 2.532 |
| IPI00759663.1 | Isoform Cytoplasmic+peroxisomal of Peroxiredoxin-5, mitochondrial | 3.491 | 4.923 | 5.620 | 3.012 | 4.002 |
| IPI00783859.2 | Isoform 2 of Vacuolar protein sorting-associated protein 13D | 4.988 | 17.839 | 25.242 | 7.435 | 2.409 |
| IPI00783987.2 | Complement C3 (Fragment) | 1.980 | 3.439 | 6.345 | 1.877 | 2.164 |
| IPI00784295.2 | Isoform 1 of Heat shock protein HSP 90-alpha | 2.433 | 2.968 | 4.964 | 1.766 | 1.961 |
| IPI00784950.1 | Putative uncharacterized protein DKFZp686L19235 | 3.468 | 2.723 | 5.483 | 1.624 | 1.930 |
| IPI00784985.1 | IGK@ protein | 2.223 | 3.329 | 4.496 | 2.052 | 2.583 |
| IPI00789134.5 | Glyceraldehyde-3-phosphate dehydrogenase | 1.926 | 2.502 | 4.779 | 1.452 | 2.574 |
| IPI00790172.5 | Keratin, type I cuticular Ha5 | 2.163 | 12.202 | 6.334 | 2.584 | 1.246 |
| IPI00792677.2 | cDNA FLJ60097, highly similar to Tubulin alpha-ubiquitous chain | 1.382 | 1.770 | 2.776 | 1.052 | 1.476 |
| IPI00793108.2 | 98 kDa protein | 2.020 | 4.890 | 11.027 | 2.183 | 2.432 |
| IPI00793296.1 | MAM domain-containing glycosylphosphatidylinositol anchor protein 2 isoform 2 | 1.221 | 4.266 | 3.150 | 1.367 | 1.463 |
| IPI00795501.3 | Low affinity immunoglobulin gamma Fc region receptor III-B | 1.571 | 0.823 | 0.820 | 1.112 | 2.117 |
| IPI00796727.1 | Uncharacterized protein | 1.214 | 1.058 | 1.307 | 1.277 | 1.278 |
| IPI00797270.4 | Isoform 1 of Triosephosphate isomerase | 1.602 | 4.407 | 9.141 | 1.604 | 1.199 |
| IPI00807400.2 | Isoform 2 of Structural maintenance of chromosomes protein 1B | 1.721 | 2.850 | 4.573 | 1.550 | 1.726 |
| IPI00815843.1 | RPL14 protein (Fragment) | 1.016 | 1.489 | 1.604 | 1.720 | 1.596 |
| IPI00816314.1 | Putative uncharacterized protein DKFZp686I15196 | 3.011 | 3.313 | 6.320 | 2.707 | 3.279 |
| IPI00816687.1 | FGB protein (Fragment) | 1.627 | 1.774 | 2.386 | 1.309 | 1.816 |
| IPI00816741.1 | Complement component 5 variant (Fragment) | 1.971 | 6.126 | 7.845 | 2.710 | 1.725 |
| IPI00829896.1 | Hemoglobin Lepore-Baltimore (Fragment) | 2.476 | 2.209 | 3.061 | 1.420 | 1.481 |
| IPI00845508.3 | BAH and coiled-coil domain-containing protein 1 | 2.207 | 4.497 | 7.543 | 2.159 | 3.719 |
| IPI00848259.1 | Merlin variant 14 | 0.763 | 3.070 | 1.933 | 2.357 | 1.915 |
| IPI00848276.1 | Isoform 1 of Uncharacterized protein C10orf18 | 0.452 | 1.711 | 1.520 | 0.635 | 0.597 |
| IPI00853045.1 | Anti-RhD monoclonal T125 kappa light chain | 2.325 | 2.720 | 4.016 | 1.949 | 3.138 |
| IPI00853068.2 | Hemoglobin alpha-2 | 46.021 | 2.885 | 11.499 | 2.248 | 3.976 |
| IPI00853525.1 | Uncharacterized protein | 1.991 | 1.067 | 1.430 | 1.674 | 2.615 |
| IPI00855985.2 | Mitogen-activated protein kinase 1 | 1.515 | 2.157 | 3.919 | 1.315 | 2.010 |
| IPI00872684.2 | cDNA FLJ54141, highly similar to Ezrin | 1.669 | 1.861 | 1.318 | 1.498 | 2.844 |
| IPI00878282.1 | 23 kDa protein | 2.295 | 6.632 | 15.416 | 2.522 | 1.777 |
| IPI00879437.1 | Protein | 1.872 | 1.883 | 2.321 | 1.522 | 2.591 |
| IPI00884996.1 | Isoform 1 of Dynein heavy chain 6, axonemal | 2.020 | 2.936 | 5.556 | 1.342 | 1.546 |
| IPI00885046.1 | Isoform 3 of Dynein heavy chain 1, axonemal | 32.735 | 12.945 | 42.874 | 3.459 | 2.779 |
| IPI00885122.1 | Isoform 1 of Diffuse panbronchiolitis critical region protein 1 | 2.041 | 3.306 | 9.940 | 1.637 | 2.026 |
| IPI00888187.2 | Putative zinc finger protein 487 | 8.799 | 4.879 | 5.499 | 3.205 | 5.113 |
| IPI00893981.1 | Uncharacterized protein | 1.361 | 2.469 | 5.074 | 1.032 | 1.852 |
| IPI00902755.1 | FGA protein (Fragment) | 2.400 | 2.028 | 3.058 | 2.061 | 2.746 |
| IPI00903112.1 | cDNA FLJ36533 fis, clone TRACH2004428, highly similar to Lactotransferrin (Fragment) | 2.664 | 3.137 | 5.644 | 2.044 | 2.428 |
| IPI00908402.1 | cDNA FLJ51275 | 1.554 | 0.515 | 0.526 | 1.250 | 2.617 |
| IPI00908776.3 | cDNA FLJ61380, highly similar to Alpha-actinin-4 | 1.971 | 2.092 | 2.061 | 1.342 | 2.370 |
| IPI00908791.3 | L-lactate dehydrogenase | 1.690 | 2.824 | 5.085 | 1.566 | 2.454 |
| IPI00908881.3 | Glucose-6-phosphate isomerase | 2.095 | 4.977 | 9.723 | 1.670 | 2.216 |
| IPI00909059.5 | cDNA FLJ53910, highly similar to Keratin, type II cytoskeletal 6A | 0.912 | 1.283 | 0.913 | 2.144 | 2.156 |
| IPI00909509.1 | cDNA FLJ59138, highly similar to Annexin A2 | 1.856 | 1.005 | 1.392 | 1.114 | 1.521 |
| IPI00909530.1 | cDNA FLJ52843, highly similar to Histone H3.3 | 1.910 | 3.080 | 6.500 | 1.568 | 2.261 |
| IPI00909658.1 | cDNA FLJ52759, highly similar to Plastin-2 | 1.812 | 2.489 | 6.118 | 1.494 | 2.698 |
| IPI00910407.1 | Peptidyl-prolyl cis-trans isomerase | 1.879 | 2.581 | 2.584 | 1.471 | 1.961 |
| IPI00910544.1 | cDNA FLJ57640, highly similar to Serpin B5 | 1.521 | 2.576 | 2.975 | 1.682 | 2.259 |
| IPI00910709.1 | cDNA FLJ53133, highly similar to Erythrocyte band 7 integral membrane protein | 1.133 | 3.066 | 0.904 | 0.868 | 1.604 |
| IPI00910754.1 | L-lactate dehydrogenase A chain isoform 2 | 1.618 | 2.749 | 4.408 | 1.422 | 2.253 |
| IPI00910974.1 | Phosphoglycerate kinase | 1.486 | 4.229 | 10.034 | 1.340 | 1.553 |
| IPI00910979.1 | pyruvate kinase isozymes M1/M2 isoform e | 1.685 | 1.671 | 2.448 | 1.421 | 2.812 |
| IPI00911039.1 | cDNA FLJ54408, highly similar to Heat shock 70 kDa protein 1 | 1.587 | 1.556 | 1.862 | 1.324 | 1.772 |
| IPI00914847.2 | nebulin isoform 1 | 0.867 | 1.322 | 1.361 | 1.031 | 1.161 |
| IPI00916185.2 | Isoform 4 of Dynein heavy chain 1, axonemal | 17.718 | 8.329 | 25.793 | 2.109 | 2.507 |
| IPI00916517.2 | 28 kDa protein | 1.997 | 3.800 | 4.818 | 1.948 | 3.008 |
| IPI00917176.1 | Isoform 5 of Dynein heavy chain 1, axonemal | 21.419 | 5.385 | 11.708 | 1.394 | 1.894 |
| IPI00922216.1 | Adenylyl cyclase-associated protein | 1.669 | 2.359 | 2.792 | 1.493 | 2.233 |
| IPI00922673.1 | cDNA FLJ55309 | 11.198 | 2.427 | 5.107 | 7.320 | 5.842 |
| IPI00924436.1 | Uncharacterized protein | 0.855 | 0.734 | 0.950 | 1.141 | 1.961 |
| IPI00924608.1 | vacuolar protein sorting-associated protein 13D isoform 1 | 4.375 | 15.621 | 22.791 | 6.247 | 2.323 |
| IPI00925547.2 | Uncharacterized protein | 2.438 | 2.568 | 4.876 | 1.556 | 1.963 |
| IPI00927887.1 | Histone H2A | 1.023 | 0.668 | 0.793 | 0.664 | 1.378 |
| IPI00930073.1 | cDNA, FLJ93744, highly similar to Homo sapiens keratin 6E (KRT6E), mRNA | 0.878 | 1.278 | 1.048 | 2.277 | 2.347 |
| IPI00930144.1 | Histone H2A | 1.101 | 0.772 | 0.843 | 0.711 | 1.325 |
| IPI00930351.1 | Hbbm fused globin protein (Fragment) | 5.696 | 2.581 | 4.831 | 1.515 | 1.644 |
| IPI00930442.1 | Putative uncharacterized protein DKFZp686M24218 | 1.532 | 1.295 | 1.792 | 1.656 | 2.465 |
| IPI00930614.1 | GUCA1B protein (Fragment) | 0.943 | 1.457 | 1.403 | 1.085 | 1.278 |
| IPI00939521.1 | 10 kDa protein | 3.468 | 2.723 | 5.483 | 1.624 | 1.930 |
| IPI00940399.2 | Uncharacterized protein | 0.596 | 0.553 | 1.176 | 0.232 | 0.414 |
| IPI00940673.2 | cDNA FLJ36348 fis, clone THYMU2007025, highly similar to TRANSKETOLASE | 2.356 | 2.516 | 4.369 | 1.762 | 2.641 |
| IPI00945626.2 | cDNA FLJ54029, highly similar to Serotra nsferrin | 2.124 | 1.674 | 2.181 | 1.819 | 2.673 |
| IPI00946655.1 | Isoform 1 of Actin-related protein 3C | 3.030 | 8.158 | 11.061 | 3.034 | 2.477 |
| IPI00947307.1 | cDNA FLJ58075, highly similar to Ceruloplasmin | 3.799 | 2.779 | 4.874 | 2.259 | 4.018 |
| IPI00964070.1 | Uncharacterized protein | 2.290 | 4.252 | 7.797 | 1.974 | 2.200 |
| IPI00964635.1 | Uncharacterized protein | 1.843 | 1.324 | 1.650 | 1.286 | 1.907 |
| IPI00965713.3 | fibrinogen beta chain isoform 2 preproprotein | 1.488 | 1.582 | 2.102 | 1.172 | 1.673 |
| IPI00966664.1 | Uncharacterized protein | 2.641 | 1.602 | 2.256 | 1.275 | 2.020 |
| IPI00967416.1 | Uncharacterized protein | 2.167 | 1.776 | 2.853 | 1.556 | 2.101 |
| IPI00967791.1 | Protein | 1.582 | 2.155 | 2.671 | 1.559 | 2.464 |
| IPI00972963.1 | Lambda light chain of human immunoglobulin surface antigen-related protein (Fragment) | 2.264 | 2.955 | 4.213 | 2.075 | 3.259 |
| IPI00973588.1 | Full-length cDNA clone CS0DI019YF20 of Placenta of Homo sapiens (Fragment) | 2.706 | 9.806 | 15.041 | 3.359 | 2.641 |
| IPI00974274.1 | Uncharacterized protein | 1.968 | 2.167 | 2.275 | 1.697 | 2.331 |
| IPI00974428.1 | Uncharacterized protein | 55.064 | | 1.529 | 2.624 | 4.044 |
| IPI00975690.1 | Vimentin variant 3 | 2.191 | 3.482 | 6.483 | 1.417 | 1.796 |
| IPI00975801.1 | Uncharacterized protein | 1.883 | 0.863 | 0.946 | 1.047 | 2.470 |
| IPI00976599.1 | Uncharacterized protein | 1.596 | 0.743 | 1.063 | 0.907 | 3.364 |
| IPI00977575.1 | Uncharacterized protein | 39.712 | 4.075 | 22.681 | 1.709 | 1.810 |
| IPI00978296.1 | Uncharacterized protein | 28.145 | 0.255 | 1.653 | 1.637 | 2.836 |
| IPI00978338.1 | Wilms tumor 1 | 5.475 | 3.551 | 10.012 | 1.042 | 1.521 |
| IPI00978796.1 | 17 kDa protein | 1.740 | 3.718 | 5.675 | 1.139 | 2.307 |
| IPI00980674.1 | Uncharacterized protein | 13.509 | 1.040 | 2.885 | 1.677 | 2.479 |
| IPI00981317.1 | cDNA FU75025, highly similar to Homo sapiens peptidylprolyl isomerase A (cyclophilin A) (PPIA), transcript variant 2, mRNA | 2.557 | 3.259 | 4.123 | 1.803 | 2.636 |
| IPI00981659.1 | Similar to Cold agglutinin FS-1 H-chain | 1.504 | 1.967 | 3.733 | 0.982 | 1.173 |
| IPI00981943.1 | Uncharacterized protein | 2.022 | 0.885 | 1.226 | 1.067 | 1.914 |
| IPI00982472.1 | Transaldolase | 1.870 | 2.506 | 4.255 | 1.583 | 2.420 |
| IPI00984226.2 | Uncharacterized protein | 2.988 | 0.950 | 1.276 | 1.116 | 1.606 |
| IPI00985334.2 | titin isoform N2-B | 6.506 | 1.481 | 3.001 | 2.668 | 2.421 |
| IPI01009324.2 | Uncharacterized protein | 2.015 | 2.419 | 3.996 | 1.458 | 2.271 |
| IPI01009332.1 | cDNA FU61543, highly similar to Desmoplakin | 0.973 | 1.588 | 1.597 | 1.892 | 1.943 |
| IPI01009809.1 | 51 kDa protein | 1.093 | 1.572 | 1.574 | 1.323 | 1.570 |
| IPI01010323.1 | cDNA FU38286 fis, clone FCBBF3008153, highly similar to ALPHA-AMYLASE 2B | 1.005 | 0.681 | 0.714 | 0.941 | 2.218 |
| IPI01011210.1 | Isoform 4 of Potassium voltage-gated channel subfamily C member 2 | 1.865 | 4.165 | 6.680 | 1.856 | 3.342 |
| IPI01011319.1 | annexin A6 isoform 2 | 2.030 | 2.117 | 2.293 | 1.766 | 2.373 |
| IPI01011344.1 | Uncharacterized protein | 1.549 | 3.576 | 4.754 | 1.731 | 2.201 |
| IPI01011530.1 | Uncharacterized protein | 1.711 | 3.147 | 4.479 | 1.649 | 2.247 |
| IPI01011804.1 | Uncharacterized protein | 1.378 | 2.425 | 4.677 | 1.082 | 2.089 |
| IPI01011912.1 | Phosphoglycerate kinase | 1.462 | 3.359 | 8.933 | 1.331 | 1.826 |
| IPI01011970.1 | 6-phosphogluconate dehydrogenase, decarboxylating | 1.477 | 1.984 | 3.160 | 1.281 | 1.592 |
| IPI01012623.1 | Uncharacterized protein | 2.982 | 6.695 | 10.668 | 2.551 | 3.922 |
| IPI01012744.1 | Uncharacterized protein | 1.935 | 11.056 | 8.458 | 3.207 | 1.616 |
| IPI01013112.1 | Uncharacterized protein | 2.596 | 8.476 | 17.899 | 2.424 | 3.131 |
| IPI01013314.1 | cDNA FU53395, highly similar to Prolyl 3-hydroxylase 1 | 18.301 | 27.571 | 55.459 | 16.630 | 3.594 |
| IPI01013441.1 | Uncharacterized protein | 2.293 | 1.376 | 1.354 | 1.366 | 2.016 |
| IPI01013543.1 | Triosephosphate isomerase | 1.583 | 2.620 | 3.451 | 1.544 | 1.736 |
| IPI01014005.1 | AMY1A protein (Fragment) | 1.105 | 0.879 | 0.901 | 1.148 | 2.442 |
| IPI01014138.1 | Uncharacterized protein | 1.679 | 2.739 | 3.783 | 1.830 | 2.511 |
| IPI01014668.1 | Isoform 6 of Afadin | 0.392 | 0.686 | 0.648 | 0.467 | 0.630 |
| IPI01014975.1 | Uncharacterized protein | 2.447 | 1.090 | 1.955 | 1.969 | 2.527 |
| IPI01015050.2 | Uncharacterized protein | 1.539 | 2.230 | 3.161 | 1.285 | 2.343 |
| IPI01015738.1 | Uncharacterized protein | 1.962 | 2.414 | 2.212 | 1.611 | 2.282 |
| IPI01018060.1 | Ig lambda-3 chain C regions | 2.143 | 3.807 | 6.225 | 2.172 | 3.213 |
| IPI01018799.1 | Isoform 2 of Dystonin | 2.614 | 2.861 | 4.054 | 2.074 | 3.431 |
| IPI01020720.1 | cDNA FU54328, highly similar to Heat shock 70 kDa protein 1 | 2.232 | 3.358 | 5.006 | 1.677 | 2.299 |
| IPI01021999.1 | Uncharacterized protein | 1.813 | 5.475 | 6.467 | 1.970 | 1.650 |
| IPI01022175.1 | cDNA FU55805, highly similar to Keratin, type II cytoskeletal 4 | 0.807 | 1.416 | 1.443 | 1.087 | 1.078 |
| IPI01022327.1 | keratin, type II cytoskeletal 4 | 0.861 | 1.347 | 1.387 | 1.043 | 1.078 |
| IPI01025024.1 | 27 kDa protein | 1.850 | 1.722 | 2.055 | 1.495 | 2.357 |
| IPI01025103.1 | 20 kDa protein | 1.454 | 2.261 | 3.413 | 1.400 | 2.351 |
| IPI01026451.1 | Protein | 2.571 | 7.608 | 26.692 | 0.218 | 0.825 |
| IPI00019779.3 | Putative uncharacterized protein DKFZp686B0790 | 1.964 | 7.204 | 22.332 | 0.817 | 2.105 |
| Q73NE3 | ATP-dependent protease ATPase subunit HsIU OS=Treponema denticola (strain ATCC 35405 / CIP 103919 / DSM 14222) GN=hslU PE=3 SV=1 - [HSLU_TREDE] | 2.283 | 4.788 | 10.385 | 2.164 | 3.335 |
| Q97QZ6 | Putative lipid kinase SP_1045 OS=Streptococcus pneumoniae GN=SP_1045 PE=1 SV=1 - [Y1045_STRPN] | 1.346 | 2.270 | 6.525 | 0.712 | 1.261 |

**SUPPLEMENTARY TABLE 2**

| CLUSTER 3 | | |
|---|---|---|
| Sample # 52: | IPI00167191.1 | CDNA FLJ25707 fis, clone TST04879 |

| CLUSTER 4 | | |
|---|---|---|
| Sample # 4: | A8AW24 | Isoleucine--tRNA ligase OS=Streptococcus gordonii (strain Challis / ATCC 35105 / CH1 / DL1 / V288) GN=ileS PE=3 SV=1 |
| Sample # 85: | IPI00334400.2 | Isoform 2 of Plakophilin-4 |
| Sample # 163: | IPI00848276.1 | Isoform 1 of Uncharacterized protein C10orf18 |
| Sample # 209: | IPI00940399.2 | Uncharacterized protein |
| Sample # 257: | IPI01014668.1 | Isoform 6 of Afadin |

| CLUSTER 5 | | |
|---|---|---|
| Sample # 269: | IPI01026451.1 | Protein |

**SUPPLEMENTARY TABLE 3**

| CLUSTER 1C | | |
|---|---|---|
| Sample # 49: | IPI00177428.1 | Isoform 2 of Mitochondrial intermembrane space import and assembly protein 40 |
| Sample # 76: | IPI00328296.2 | PDZ domain-containing protein GIPC2 |

| CLUSTER 1D | | |
|---|---|---|
| Sample # 17: | IPI00010349.1 | Alkyldihydroxyacetonephosphate synthase, peroxisomal |
| Sample # 66: | IPI00292579.4 | Stabilin-2 |
| Sample # 99: | IPI00470476.3 | Uncharacterized protein C9orf144A |
| Sample # 125: | IPI00745280.2 | Similar to Keratin, type II cytoskeletal 7 |
| Sample # 130: | IPI00783859.2 | Isoform 2 of Vacuolar protein sorting-associated protein 13D |
| Sample # 181: | IPI00924608.1 | vacuolar protein sorting-associated protein 13D isoform 1 |

**SUPPLEMENTARY TABLE 4**

| CLUSTER 1A3 | | |
|---|---|---|
| Sample # 12: | IPI00009724.3 | Isoform 1 of EF-hand calcium-binding domain-containing protein 6 |
| Sample # 46: | IPI00168728.1 | FLJ00385 protein (Fragment) |
| Sample # 84: | IPI00418153.1 | Putative und uncharacterized protein DKFZp686I15212 |
| Sample # 103: | IPI00552637.1 | G-protein coupled receptor |
| Sample # 108: | IPI00556287.1 | Putative uncharacterized protein |
| Sample # 129: | IPI00790172.5 | Keratin, type I cuticular Ha5 |
| Sample # 183: | IPI00946655.1 | Isoform 1 of Actin-related protein 3C |
| Sample # 192: | IPI00973588.1 | Full-length cDNA clone CS0DI019YF20 of Placenta of Homo sapiens (Fragment) |
| Sample # 215: | IPI01012744.1 | Uncharacterized protein |
| Sample # 216: | IPI01013112.1 | Uncharacterized protein |

| CLUSTER 1A4 | | |
|---|---|---|
| Sample # 74: | IPI00375293.2 | Isoform 2 of High affinity immunoglobulin gamma Fc receptor I |
| Sample # 232: | O83773 | Uncharacterized protein TP_0795 OS=Treponema pallidum (strain Nichols) GN=TP_0795 PE=4 SV=1 - [Y795_TREPA] |

**SUPPLEMENTARY TABLE 5**

| Accession | Description | Number of quant peptides | A6: 114/113 | A6: 115/113 | A6: 116/113 | A6: 117/113 | A6: 118/113 | A6: 119/113 |
|---|---|---|---|---|---|---|---|---|
| A7I3U7 | Elongation factor Tu OS=Campylobacter hominis (strain ATCC BAA-381 / LMG 19568 / NCTC 13146 / CH001A) GN=tuf PE=3 SV=1 - [EFTU_CAMHC] | 1 | 1.334 | 1.068 | 1.383 | 0.931 | 0.965 | 0.909 |
| IPI00002557.1 | Coatomer subunit gamma-2 | 3 | 2.422 | 3.431 | 5.719 | 1.270 | 1.857 | 0.973 |
| IPI00002851.1 | Cystatin-D | 45 | 1.067 | 1.155 | 1.212 | 1.946 | 1.669 | 2.013 |
| IPI00003269.1 | Beta-actin-like protein 2 | 1 | 1.133 | 1.512 | 1.605 | 1.476 | 1.238 | 1.541 |
| IPI00003935.6 | Histone H2B type 2-E | 5 | 1.265 | 1.484 | 1.633 | 0.388 | 0.634 | 0.551 |
| IPI00004573.2 | Polymeric immunoglobulin receptor | 344 | 1.355 | 1.004 | 1.141 | 1.339 | 1.164 | 2.048 |
| IPI00004656.3 | Beta-2-microglobulin | 12 | 1.151 | 0.818 | 1.107 | 1.181 | 0.921 | 1.456 |
| IPI00005721.1 | Neutrophil defensin 1 | 31 | 1.339 | 1.818 | 3.735 | 1.321 | 1.880 | 0.325 |
| IPI00007047.1 | Protein S100-A8 | 105 | 2.089 | 3.485 | 5.821 | 3.001 | 3.414 | 0.761 |
| IPI00007797.3 | Fatty acid-binding protein, epidermal | 37 | 0.869 | 0.659 | 1.116 | 0.868 | 0.730 | 1.118 |
| IPI00008405.5 | Arylsulfatase F | 1 | 2.459 | 1.617 | 2.073 | 1.893 | 1.433 | 1.685 |
| IPI00008895.1 | Epithelial membrane protein 2 | 2 | 1.432 | 1.014 | 1.025 | 0.465 | 0.654 | 0.343 |
| IPI00009650.1 | Lipocalin-1 | 88 | 0.821 | 0.659 | 0.774 | 0.770 | 0.849 | 1.003 |
| IPI00010182.4 | Isoform 1 of Acyl-CoA-binding protein | 2 | 0.541 | 0.683 | 1.343 | 0.535 | 0.586 | 1.083 |
| IPI00010471.6 | Plastin-2 | 60 | 1.927 | 2.427 | 3.986 | 1.449 | 1.981 | 0.749 |
| IPI00010796.1 | Protein disulfide-isomerase | 22 | 1.158 | 1.242 | 1.913 | 1.246 | 1.360 | 1.128 |
| IPI00010896.3 | Chloride intracellular channel protein 1 | 1 | 1.302 | 2.597 | 4.398 | 1.171 | 1.693 | 0.000 |
| IPI00012024.1 | Histatin-1 | 19 | 1.681 | 2.703 | 1.813 | 8.008 | 3.163 | 5.536 |
| IPI00012199.1 | Coiled-coil domain-containing protein 86 | 5 | 3.494 | 1.199 | 0.471 | 2.583 | 1.608 | 9.492 |
| IPI00012525.1 | Putative uncharacterized protein (Fragment) | 3 | 0.923 | 1.037 | 0.621 | 1.198 | 0.931 | 2.992 |
| IPI00012796.1 | Glutamate decarboxylase 2 | 6 | 0.524 | 0.591 | 0.909 | 0.338 | 0.252 | 0.342 |
| IPI00013382.1 | Cystatin-SA | 154 | 1.612 | 2.447 | 1.976 | 3.388 | 3.313 | 2.284 |
| IPI00013885.1 | Caspase-14 | 4 | 0.821 | 0.982 | 1.293 | 0.866 | 1.008 | 1.908 |
| IPI00013890.2 | Isoform 1 of 14-3-3 protein sigma | 2 | 0.749 | 0.478 | 0.614 | 0.806 | 0.626 | 1.215 |
| IPI00013895.1 | Protein S100-A11 | 3 | 0.964 | 0.923 | 2.966 | 0.659 | 0.985 | 1.115 |
| IPI00016347.5 | Isoform 3 of Uncharacterized protein C2orf54 | 1 | 4.154 | 8.928 | 16.772 | 1.275 | 2.398 | 1.915 |
| IPI00017526.1 | Protein S100-P | 8 | 1.552 | 2.065 | 4.222 | 1.358 | 1.880 | 0.603 |
| IPI00017672.4 | cDNA FLJ25678 fis, clone TST04067, highly similar to PURINE NUCLEOSIDE PHOSPHORYLASE | 5 | 0.918 | 1.218 | 5.022 | 1.120 | 1.446 | 0.815 |
| IPI00019038.1 | Lysozyme C | 4 | 2.326 | 1.456 | 2.240 | 1.721 | 1.671 | 3.324 |
| IPI00019449.1 | Non-secretory ribonuclease | 2 | 1.376 | 2.430 | 3.652 | 1.226 | 1.337 | 1.147 |
| IPI00019502.3 | Isoform 1 of Myosin-9 | 2 | 2.000 | 3.924 | 8.217 | 1.892 | 3.411 | 0.808 |
| IPI00020008.1 | NEDD8 | 3 | 1.428 | 1.588 | 2.203 | 1.005 | 1.461 | 1.352 |
| IPI00020091.1 | Alpha-1-acid glycoprotein 2 | 17 | 1.535 | 1.385 | 4.699 | 1.421 | 1.782 | 1.147 |
| IPI00021085.1 | Peptidoglycan recognition protein 1 | 5 | 1.221 | 1.458 | 2.897 | 0.970 | 1.046 | 0.753 |
| IPI00021263.3 | 14-3-3 protein zeta/delta | 3 | 1.178 | 1.027 | 1.446 | 1.289 | 1.279 | 1.287 |
| IPI00021304.1 | Keratin, type II cytoskeletal 2 epidermal | 5 | 0.982 | 1.009 | 1.276 | 1.151 | 3.128 | 1.063 |
| IPI00021447.1 | Alpha-amylase 2B | 3 | 1.079 | 0.862 | 0.705 | 1.222 | 0.933 | 2.067 |
| IPI00021828.1 | Cystatin-B | 81 | 0.828 | 0.544 | 0.876 | 0.676 | 0.686 | 1.099 |
| IPI00021841.1 | Apolipoprotein A-I | 18 | 0.835 | 0.832 | 1.867 | 0.694 | 0.860 | 0.745 |
| IPI00022429.3 | Alpha-1-acid glycoprotein 1 | 53 | 1.424 | 1.537 | 4.423 | 1.523 | 1.798 | 0.917 |
| IPI00022463.2 | Serotransferrin | 146 | 1.459 | 1.092 | 2.155 | 1.029 | 1.126 | 0.881 |
| IPI00022488.1 | Hemopexin | 32 | 1.404 | 1.403 | 2.394 | 0.981 | 1.138 | 0.948 |
| IPI00022974.1 | Prolactin-inducible protein | 246 | 1.227 | 1.209 | 1.292 | 1.748 | 1.622 | 1.103 |
| IPI00022990.1 | Statherin | 7 | 1.429 | 2.406 | 2.568 | 3.207 | 2.496 | 2.384 |
| IPI00023011.2 | Submaxillary gland androgen-regulated protein 3B | 90 | 1.742 | 2.454 | 2.287 | 2.367 | 2.098 | 2.577 |
| IPI00023038.2 | Basic salivary proline-rich protein 1 | 4 | 1.218 | 0.961 | 1.368 | 1.927 | 1.498 | 3.110 |
| IPI00027462.1 | Protein S100-A9 | 96 | 1.798 | 3.205 | 6.274 | 2.954 | 3.150 | 1.038 |
| IPI00027463.1 | Protein S100-A6 | 2 | 2.194 | 2.125 | 3.066 | 1.586 | 2.024 | 1.628 |
| IPI00027509.5 | Matrix metalloproteinase-9 | 10 | 1.467 | 1.875 | 4.619 | 1.046 | 1.606 | 0.603 |
| IPI00027769.1 | Neutrophil elastase | 12 | 1.439 | 2.081 | 3.690 | 0.986 | 1.648 | 0.671 |
| IPI00028064.1 | Cathepsin G | 2 | 1.820 | 1.721 | 2.885 | 1.107 | 2.606 | 0.827 |
| IPI00028931.2 | Desmoglein-2 | 5 | 0.928 | 1.331 | 0.424 | 0.680 | 0.401 | 1.298 |
| IPI00031983.4 | UDP-GlcNAc:betaGal beta-1,3-N-acetylglucosaminyltransfera se 3 | 1 | 1.175 | 1.644 | 1.973 | 1.315 | 1.081 | 1.234 |
| IPI00032220.3 | Angiotensinogen | 3 | 1.522 | 0.794 | 2.030 | 0.934 | 1.162 | 1.027 |
| IPI00032293.1 | Cystatin-C | 25 | 1.258 | 1.117 | 1.166 | 1.533 | 1.176 | 1.776 |
| IPI00032294.1 | Cystatin-S | 234 | 1.385 | 1.513 | 1.441 | 2.037 | 1.440 | 1.319 |
| IPI00037070.3 | Uncharacterized protein | 1 | 0.603 | 0.706 | 3.183 | 0.527 | 0.552 | 0.625 |
| IPI00060800.5 | Zymogen granule protein 16 homolog B | 154 | 2.141 | 1.224 | 0.812 | 1.888 | 1.294 | 0.914 |
| IPI00065475.6 | Isoform 2 of UPF0585 protein C16orf13 | 1 | 1.307 | 1.489 | 2.017 | 1.064 | 0.979 | 1.041 |
| IPI00103636.1 | Isoform 2 of WAP four-disulfide core domain protein 2 | 11 | 1.213 | 0.761 | 1.013 | 1.286 | 1.328 | 2.436 |
| IPI00141938.4 | histone H2A.V isoform 2 | 7 | 1.184 | 1.149 | 1.390 | 0.377 | 0.633 | 0.822 |
| IPI00152154.2 | Mucin-7 | 11 | 1.390 | 1.341 | 1.362 | 1.481 | 1.205 | 0.846 |
| IPI00166729.4 | Zinc-alpha-2-glycoprotein | 126 | 1.184 | 0.986 | 1.046 | 1.204 | 1.017 | 1.645 |
| IPI00169244.1 | 106 kDa protein | 1 | 1.285 | 1.692 | 1.105 | 1.831 | 1.341 | 2.026 |
| IPI00171196.2 | Isoform 3 of Keratin, type I cytoskeletal 13 | 28 | 0.997 | 2.372 | 3.765 | 0.914 | 0.921 | 0.784 |
| IPI00174541.1 | Isoform 4 of Interleukin-1 receptor antagonist protein | 27 | 0.951 | 0.847 | 0.969 | 0.874 | 0.848 | 1.522 |
| IPI00178926.2 | Immunoglobulin J chain | 48 | 1.411 | 0.946 | 1.422 | 1.191 | 1.292 | 1.833 |
| IPI00180240.2 | Thymosin beta-4-like protein 3 | 4 | 1.890 | 2.250 | 5.361 | 1.801 | 2.320 | 1.482 |
| IPI00182138.4 | Isoform 2 of Granulins | 8 | 1.159 | 1.156 | 2.207 | 1.104 | 1.212 | 0.833 |
| IPI00215628.1 | Isoform V1 of Versican core protein | 4 | 0.753 | 0.233 | 0.118 | 0.868 | 0.167 | 2.209 |
| IPI00216298.6 | Thioredoxin | 20 | 0.714 | 0.530 | 0.903 | 0.637 | 0.618 | 0.868 |
| IPI00216691.5 | Profilin-1 | 43 | 2.306 | 2.912 | 7.249 | 1.671 | 2.447 | 0.862 |
| IPI00216835.2 | Isoform 2 of NADPH oxidase activator 1 | 2 | 4.163 | 3.228 | 1.127 | 3.132 | 3.034 | 4.653 |
| IPI00217473.5 | Hemoglobin subunit zeta | 1 | 2.065 | 4.113 | 2.853 | 5.036 | 4.045 | 3.416 |
| IPI00217846.3 | Isoform 4 of Uncharacterized protein C5orf25 | 6 | 1.208 | 1.328 | 2.126 | 1.044 | 0.906 | 1.302 |
| IPI00217963.3 | Keratin, type I cytoskeletal 16 | 1 | 1.136 | 1.173 | 1.859 | 1.552 | 1.383 | 0.770 |
| IPI00217966.9 | Isoform 1 of L-lactate dehydrogenase A chain | 7 | 1.420 | 1.287 | 3.600 | 1.170 | 1.608 | 1.021 |
| IPI00218131.3 | Protein S100-A12 | 7 | 2.018 | 2.382 | 5.247 | 1.969 | 3.303 | 0.870 |
| IPI00218918.5 | Annexin A1 | 6 | 0.901 | 0.866 | 1.135 | 0.842 | 1.134 | 1.151 |
| IPI00219018.7 | Glyceraldehyde- 3-phosphate dehydrogenase | 7 | 1.890 | 2.015 | 3.068 | 2.405 | 3.303 | 1.254 |
| IPI00219365.3 | Moesin | 5 | 2.446 | 3.238 | 5.521 | 1.827 | 2.468 | 1.272 |
| IPI00219757.1 3 | Glutathione S-transferase P | 5 | 1.167 | 1.143 | 2.071 | 0.961 | 1.121 | 1.127 |
| IPI00220327.4 | Keratin, type II cytoskeletal 1 | 10 | 1.379 | 1.390 | 1.249 | 1.338 | 2.501 | 1.922 |
| IPI00221362.3 | Isoform 3 of Apoptosis-associated speck-like protein containing a CARD | 3 | 1.298 | 1.275 | 2.918 | 0.872 | 1.230 | 0.674 |
| IPI00236554.1 | Isoform H14 of Myeloperoxidase | 7 | 0.972 | 1.284 | 1.511 | 0.698 | 0.702 | 0.622 |
| IPI00255103.8 | Isoform 3 of Contactin-associated protein-like 3B | 1 | 0.804 | 0.796 | 1.113 | 0.715 | 0.650 | 0.736 |
| IPI00290077.3 | Keratin, type I cytoskeletal 15 | 3 | 0.713 | 1.001 | 1.373 | 0.894 | 1.092 | 0.636 |
| IPI00291410.3 | Isoform 1 of Long palate, lung and nasal epithelium carcinoma-associated protein 1 | 5 | 0.565 | 0.499 | 0.630 | 0.648 | 0.723 | 0.588 |
| IPI00291922.2 | Proteasome subunit alpha type-5 | 2 | 1.205 | 1.447 | 3.311 | 1.219 | 1.520 | 0.848 |
| IPI00293276.1 0 | Macrophage migration inhibitory factor | 2 | 1.102 | 1.154 | 3.849 | 0.903 | 1.245 | 0.864 |
| IPI00295105.3 | cDNA FU60163, highly similar to Carbonic anhydrase 6 | 89 | 1.200 | 0.995 | 0.740 | 1.345 | 0.973 | 1.944 |
| IPI00296654.2 | Bactericidal/permeability-increasing protein-like 1 | 102 | 1.038 | 0.964 | 1.128 | 1.036 | 1.059 | 0.979 |
| IPI00298497.3 | Fibrinogen beta chain | 3 | 1.271 | 1.495 | 2.748 | 1.090 | 1.251 | 0.768 |
| IPI00299078.1 | Salivary acidic proline-rich phosphoprotein 1/2 | 105 | 1.717 | 1.175 | 0.948 | 1.780 | 0.958 | 2.336 |
| IPI00299547.4 | Isoform 1 of Neutrophil gelatinase-associated lipocalin | 10 | 1.335 | 1.374 | 4.163 | 0.862 | 1.261 | 1.122 |
| IPI00299729.4 | Transcobalamin-1 | 11 | 1.254 | 1.092 | 1.409 | 1.080 | 0.911 | 1.048 |
| IPI00300376.5 | Protein-glutamine gamma-glutamyltransferase E | 4 | 1.381 | 1.362 | 1.519 | 0.934 | 1.308 | 1.472 |
| IPI00300786.1 | Alpha-amylase 1 | 1385 | 1.018 | 0.914 | 0.835 | 1.024 | 0.877 | 2.316 |
| IPI00301058.5 | Vasodilator-sti mulated phosphoprotein | 6 | 1.831 | 2.325 | 3.191 | 1.358 | 1.822 | 1.087 |
| IPI00301658.7 | Isoform 3 of Protein FAM194A | 2 | 1.908 | 1.390 | 1.034 | 0.892 | 2.194 | 1.993 |
| IPI00304557.2 | Short palate, lung and nasal epithelium carcinoma-associated protein 2 | 117 | 0.967 | 0.839 | 0.582 | 1.462 | 1.133 | 1.411 |
| IPI00304808.4 | Isoform 1 of Kallikrein-1 | 33 | 1.143 | 1.071 | 1.088 | 1.193 | 0.996 | 2.770 |
| IPI00305477.6 | Cystatin-SN | 328 | 1.029 | 1.165 | 1.402 | 1.480 | 1.224 | 1.340 |
| IPI00373937.3 | Supra basin | 3 | 1.089 | 1.447 | 1.976 | 1.499 | 1.271 | 1.150 |
| IPI00374315.1 | UPF0762 protein C6orf58 | 35 | 1.219 | 0.889 | 0.891 | 0.899 | 0.814 | 0.628 |
| IPI00375307.2 | peroxiredoxin-5, mitochondrial isoform c precursor | 1 | 2.176 | 1.892 | 2.076 | 2.669 | 2.607 | 2.187 |
| IPI00377122.4 | Isoform 2 of WD repeat-containing protein KIAA1875 | 2 | 3.250 | 8.184 | 9.606 | 1.741 | 5.015 | 1.041 |
| IPI00383627.1 | Pituitary tumor transforming gene protein | 1 | 1.664 | 1.085 | 0.789 | 2.088 | 1.348 | 3.280 |
| IPI00383981.3 | AZU1 protein (Fragment) | 1 | 1.239 | 1.215 | 1.506 | 1.029 | 1.043 | 1.587 |
| IPI00384251.1 | Isoform 2 of Guanine nucleotide exchange factor for Rab-3A | 2 | 2.806 | 4.222 | 2.254 | 2.408 | 1.972 | 0.903 |
| IPI00384382.1 | AngRem52 | 2 | 5.427 | 4.310 | 0.488 | 5.921 | 5.682 | 13.414 |
| IPI00384444.6 | Keratin, type I cytoskeletal 14 | 9 | 0.828 | 1.020 | 1.545 | 1.135 | 1.213 | 0.629 |
| IPI00384975.4 | Uncharacterized protein | 3 | 1.703 | 1.882 | 3.972 | 1.491 | 1.887 | 1.072 |
| IPI00385252.1 | Ig kappa chain V-III region GOL | 1 | 1.122 | 1.376 | 1.627 | 0.603 | 0.797 | 0.991 |
| IPI00386132.1 | Ig kappa chain V-IV region JI | 3 | 1.135 | 1.470 | 2.104 | 1.164 | 1.192 | 1.304 |
| IPI00386879.1 | cDNA FLJ14473 fis, clone MAMMA1001080, highly similar to Homo sapiens SNC73 protein (SNC73) mRNA | 35 | 1.468 | 0.875 | 1.253 | 1.497 | 1.229 | 2.044 |
| IPI00397768.5 | Isoform 2 of Ribonucleoprotein PTB-binding 2 | 2 | 1.697 | 1.040 | 0.494 | 0.974 | 0.716 | 0.626 |
| IPI00399007.7 | Putative uncharacterized protein DKFZp686I04196 (Fragment) | 22 | 1.366 | 1.235 | 3.744 | 1.003 | 1.606 | 0.685 |
| IPI00399260.2 | basic salivary proline-rich protein 1 isoform 3 preproprotein | 4 | 0.817 | 0.602 | 0.858 | 1.338 | 0.896 | 2.761 |
| IPI00410714.5 | Hemoglobin subunit alpha | 295 | 3.564 | 5.378 | 11.480 | 1.052 | 1.813 | 1.174 |
| IPI00411765.3 | Isoform 2 of 14-3-3 protein sigma | 1 | 0.669 | 0.409 | 0.557 | 0.667 | 0.485 | 1.131 |
| IPI00414909.1 | Alpha-N-acetylgalactosaminidase | 2 | 2.859 | 3.040 | 0.525 | 3.474 | 2.633 | 17.162 |
| IPI00419215.6 | Alpha-2-macroglobulin-like protein 1 | 6 | 0.874 | 0.695 | 1.359 | 0.997 | 0.806 | 1.642 |
| IPI00419585.9 | Peptidyl-prolyl cis-trans isomerase A | 14 | 1.235 | 1.302 | 2.881 | 0.983 | 1.272 | 0.902 |
| IPI00423460.3 | Putative uncharacterized protein DKFZp686G21220 (Fragment) | 68 | 1.253 | 0.886 | 1.122 | 1.420 | 1.036 | 2.031 |
| IPI00426051.3 | Putative uncharacterized protein DKFZp686C15213 | 6 | 1.301 | 1.325 | 3.380 | 1.042 | 1.585 | 0.771 |
| IPI00431645.2 | 31 kDa protein | 19 | 1.534 | 1.353 | 3.752 | 1.022 | 1.684 | 1.119 |
| IPI00448925.6 | 44 kDa protein | 39 | 1.816 | 1.994 | 4.028 | 1.449 | 1.637 | 1.038 |
| IPI00451401.3 | Isoform 2 of Triosephosphate isomerase | 11 | 0.939 | 0.943 | 1.929 | 0.911 | 0.914 | 0.938 |
| IPI00453473.6 | Histone H4 | 19 | 0.940 | 1.178 | 1.774 | 0.294 | 0.442 | 0.599 |
| IPI00465248.5 | Isoform alpha-enolase of Alpha-enolase | 77 | 1.577 | 1.636 | 3.750 | 1.376 | 1.676 | 1.053 |
| IPI00465436.4 | Catalase | 3 | 0.849 | 0.892 | 4.163 | 0.689 | 0.719 | 0.588 |
| IPI00465439.5 | Fructose-bisphosphate aldolase A | 8 | 1.126 | 1.108 | 2.388 | 0.895 | 1.034 | 0.932 |
| IPI00472974.2 | PR domain zinc finger protein 2 isoform c | 1 | 1.322 | 0.782 | 0.594 | 0.499 | 0.606 | 0.826 |
| IPI00473011.3 | Hemoglobin subunit delta | 10 | 2.137 | 4.652 | 11.181 | 0.891 | 1.396 | 1.521 |
| IPI00477265.2 | archaemetzincin-2 isoform 2 | 2 | 1.045 | 1.803 | 1.409 | 1.232 | 1.202 | 1.036 |
| IPI00478003.3 | Alpha-2-macroglobulin | 32 | 1.781 | 1.541 | 2.997 | 1.162 | 1.369 | 1.261 |
| IPI00478493.3 | haptoglobin isoform 2 preproprotein | 22 | 1.618 | 1.346 | 3.690 | 1.046 | 1.698 | 1.126 |
| IPI00479186.7 | Isoform M2 of Pyruvate kinase isozymes M1/M2 | 9 | 1.071 | 1.140 | 4.945 | 1.178 | 1.376 | 0.947 |
| IPI00479708.6 | Full-length cDNA clone CS0DD006YL02 of Neuroblastoma of Homo sapiens | 25 | 1.453 | 1.136 | 1.956 | 1.318 | 1.367 | 1.145 |
| IPI00549413.2 | Uncharacterized protein | 7 | 0.823 | 0.790 | 0.992 | 0.864 | 1.115 | 1.100 |
| IPI00550731.2 | Putative uncharacterized protein | 162 | 1.388 | 1.056 | 1.827 | 1.336 | 1.377 | 1.778 |
| IPI00552432.3 | Basic salivary proline-rich protein 2 | 13 | 1.468 | 1.308 | 2.302 | 2.485 | 2.204 | 4.018 |
| IPI00552768.1 | Uncharacterized protein | 44 | 0.725 | 0.542 | 0.941 | 0.661 | 0.638 | 0.878 |
| IPI00553177.1 | Isoform 1 of Alpha-1-antitrypsin | 1 | 2.622 | 1.595 | 2.982 | 3.246 | 2.000 | 9.201 |
| IPI00554696.2 | Uncharacterized protein | 141 | 1.153 | 0.946 | 0.733 | 1.287 | 0.901 | 1.893 |
| IPI00555812.5 | vitamin D-binding protein isoform 1 precursor | 2 | 1.540 | 0.864 | 1.313 | 0.642 | 0.864 | 0.776 |
| IPI00640006.1 | rab GDP dissociation inhibitor beta isoform 2 | 2 | 1.094 | 1.139 | 2.142 | 0.875 | 0.960 | 1.079 |
| IPI00640335.1 | Protein | 2 | 1.020 | 1.000 | 1.104 | 0.715 | 0.763 | 1.381 |
| IPI00641047.5 | Uncharacterized protein | 2 | 1.030 | 1.256 | 2.841 | 0.875 | 1.109 | 0.724 |
| IPI00641737.2 | Haptoglobin | 9 | 1.558 | 1.456 | 3.624 | 1.039 | 1.631 | 1.146 |
| IPI00642247.1 | Uncharacterized protein | 1 | 1.345 | 1.142 | 1.335 | 1.592 | 1.976 | 3.355 |
| IPI00642414.1 | Adenylyl cyclase-associated protein | 8 | 1.525 | 1.749 | 4.087 | 1.080 | 1.601 | 0.632 |
| IPI00643231.1 | Uncharacterized protein | 7 | 0.799 | 0.724 | 0.856 | 0.806 | 0.949 | 1.048 |
| IPI00645363.2 | Putative uncharacterized protein DKFZp686P15220 | 13 | 1.731 | 1.816 | 3.774 | 1.500 | 1.768 | 1.103 |
| IPI00646265.2 | 58 kDa protein | 1 | 0.446 | 0.419 | 0.711 | 0.756 | 0.440 | 2.129 |
| IPI00654755.3 | Hemoglobin subunit beta | 206 | 5.138 | 7.744 | 19.343 | 0.958 | 2.207 | 1.176 |
| IPI00658053.1 | Uncharacterized protein | 3 | 1.025 | 0.706 | 1.138 | 1.215 | 1.057 | 1.976 |
| IPI00658218.1 | Isoform 5 of Deleted in malignant brain tumors 1 protein | 33 | 1.134 | 0.848 | 1.072 | 1.484 | 1.104 | 2.882 |
| IPI00719452.1 | IGL@ protein | 3 | 1.619 | 1.410 | 2.306 | 1.524 | 1.564 | 1.899 |
| IPI00735451.4 | Immunolgoobulin heavy chain | 3 | 1.458 | 1.342 | 1.931 | 1.375 | 1.529 | 1.614 |
| IPI00740545.1 | POTE ankyrin domain family member I | 1 | 1.544 | 1.863 | 5.939 | 1.646 | 1.829 | 1.201 |
| IPI00742775.1 | Bardet-Biedl syndrome 10 protein | 3 | 1.797 | 1.054 | 0.649 | 1.610 | 1.479 | 3.659 |
| IPI00745872.2 | Isoform 1 of Serum albumin | 1199 | 1.253 | 1.073 | 1.906 | 0.893 | 1.020 | 0.693 |
| IPI00748022.2 | Actin-like protein (Fragment) | 3 | 0.658 | 0.450 | 15.108 | 0.904 | 1.215 | 0.342 |
| IPI00748184.4 | Uncharacterized protein | 1 | 1.190 | 0.580 | 0.806 | 0.721 | 0.783 | 1.577 |
| IPI00759663.1 | Isoform Cytoplasmic+peroxisomal of Peroxiredoxin-5, mitochondrial | 3 | 2.019 | 1.866 | 2.939 | 2.017 | 2.349 | 1.436 |
| IPI00759832.1 | Isoform Short of 14-3-3 protein beta/alpha | 4 | 1.613 | 1.872 | 2.478 | 1.732 | 2.004 | 1.079 |
| IPI00782983.3 | Immunglobulin heavy chain variable region | 1 | 1.274 | 0.966 | 1.380 | 0.984 | 1.124 | 1.435 |
| IPI00783192.1 | Isoform 2 of Lysophospholipid acyltransferase LPCAT4 | 2 | 1.835 | 2.502 | 4.115 | 0.957 | 1.295 | 0.426 |
| IPI00783287.1 | Immunglobulin heavy chain variable region (Fragment) | 6 | 1.370 | 1.178 | 1.908 | 1.273 | 1.351 | 1.855 |
| IPI00783987.2 | Complement C3 (Fragment) | 31 | 1.310 | 1.335 | 1.824 | 1.083 | 1.300 | 0.939 |
| IPI00784430.5 | Ig kappa chain V-III region VG (Fragment) | 3 | 1.432 | 1.606 | 2.025 | 1.687 | 1.269 | 1.802 |
| IPI00784830.1 | cDNA FU41981 fis, clone SMINT2011888, highly similar to Protein Tro alphal H,myeloma | 20 | 1.313 | 0.969 | 1.755 | 1.137 | 1.364 | 1.729 |
| IPI00784842.1 | Putative uncharacterized protein DKFZp686G11190 | 5 | 1.677 | 1.158 | 2.049 | 1.110 | 1.290 | 1.805 |
| IPI00784950.1 | Putative uncharacterized protein DKFZp686L19235 | 34 | 1.385 | 1.031 | 1.794 | 1.307 | 1.680 | 1.966 |
| IPI00784985.1 | IGK@ protein | 4 | 1.659 | 1.619 | 2.137 | 1.341 | 1.216 | 1.390 |
| IPI00789337.4 | cDNA, FU79516, highly similar to 14-3-3 protein zeta/delta | 11 | 1.014 | 0.979 | 1.408 | 1.077 | 1.151 | 1.104 |
| IPI00793319.1 | Uncharacterized protein | 15 | 1.056 | 0.817 | 1.892 | 0.803 | 0.955 | 1.135 |
| IPI00793812.3 | Isoform 3 of Leukotriene A-4 hydrolase | 2 | 2.073 | 3.756 | 2.090 | 2.556 | 3.836 | 1.378 |
| IPI00795257.3 | Glyceraldehyde-3-phosphate dehydrogenase | 20 | 1.906 | 2.414 | 4.423 | 2.475 | 3.309 | 1.095 |
| IPI00796776.2 | cDNA FLJ54081, highly similar to Keratin, type II cytoskeletal 5 | 8 | 0.956 | 1.653 | 2.132 | 1.055 | 1.262 | 0.895 |
| IPI00796823.1 | Uncharacterized protein | 3 | 1.332 | 1.018 | 1.531 | 1.274 | 1.060 | 1.634 |
| IPI00797270.4 | Isoform 1 of Triosephosphate isomerase | 12 | 0.954 | 0.958 | 1.977 | 0.942 | 0.936 | 0.954 |
| IPI00807428.1 | Putative uncharacterized protein | 11 | 1.663 | 1.256 | 2.096 | 1.521 | 1.622 | 2.108 |
| IPI00816687.1 | FGB protein (Fragment) | 2 | 1.256 | 1.455 | 2.629 | 1.158 | 1.171 | 0.861 |
| IPI00829697.1 | 13 kDa protein | 2 | 1.291 | 1.053 | 1.273 | 1.258 | 1.076 | 1.344 |
| IPI00844600.1 | 9 kDa protein | 34 | 1.819 | 1.908 | 4.485 | 1.795 | 3.045 | 0.855 |
| IPI00847989.3 | Pyruvate kinase | 7 | 1.034 | 1.075 | 5.892 | 1.073 | 1.314 | 0.992 |
| IPI00853525.1 | Uncharacterized protein | 32 | 0.974 | 0.952 | 2.041 | 0.787 | 0.978 | 0.740 |
| IPI00854743.1 | Immunglobulin heavy chain variable region | 3 | 1.793 | 1.577 | 2.106 | 1.672 | 1.477 | 1.621 |
| IPI00869004.1 | Isoform 3 of Alpha-1-antitrypsin | 3 | 2.465 | 1.626 | 3.011 | 3.218 | 2.013 | 8.595 |
| IPI00872278.1 | Isoform 7 of Deleted in malignant brain tumors 1 protein | 73 | 1.096 | 0.859 | 1.032 | 1.459 | 1.193 | 2.904 |
| IPI00876888.1 | cDNA FLJ78387 | 24 | 1.938 | 2.052 | 4.124 | 1.445 | 1.739 | 1.089 |
| IPI00878551.2 | cDNA FLJ59430, highly similar to Protein disulfide-isomerase | 6 | 1.132 | 1.179 | 1.789 | 1.162 | 1.248 | 1.130 |
| IPI00879084.2 | Uncharacterized protein | 2 | 0.869 | 0.734 | 1.657 | 0.639 | 0.844 | 0.847 |
| IPI00879437.1 | Protein | 6 | 1.140 | 1.244 | 2.007 | 1.342 | 1.500 | 1.118 |
| IPI00879438.1 | Uncharacterized protein | 7 | 1.599 | 1.349 | 2.028 | 1.286 | 1.446 | 1.754 |
| IPI00883885.3 | PRB3 protein | 2 | 1.958 | 4.184 | 10.696 | 4.152 | 5.160 | 3.582 |
| IPI00884451.2 | basic salivary proline-rich protein 4 precursor | 4 | 1.487 | 1.901 | 1.782 | 3.621 | 2.533 | 6.502 |
| IPI00892657.1 | Protein | 3 | 1.054 | 0.900 | 0.852 | 0.988 | 0.843 | 1.529 |
| IPI00893981.1 | Uncharacterized protein | 15 | 1.884 | 3.572 | 9.128 | 1.543 | 2.690 | 0.933 |
| IPI00902602.1 | cDNA FLJ33251 fis, clone ASTRO2005242, highly similar to Rho guanine nucleotide exchange factor 5 | 2 | 0.931 | 0.313 | 0.160 | 1.079 | 0.232 | 2.915 |
| IPI00902755.1 | FGA protein (Fragment) | 13 | 1.306 | 1.305 | 2.165 | 0.863 | 1.298 | 0.851 |
| IPI00903112.1 | cDNA FLJ36533 fis, clone TRACH2004428, highly similar to Lactotransferrin (Fragment) | 35 | 1.075 | 1.133 | 1.275 | 0.905 | 0.900 | 1.574 |
| IPI00903245.1 | cDNA FLJ44586 fis, clone ASTRO2015162, highly similar to Choline transporter-like protein 2 | 3 | 1.185 | 1.237 | 3.194 | 0.543 | 1.024 | 0.603 |
| IPI00908402.1 | cDNA FLJ51275 | 21 | 0.875 | 0.775 | 1.042 | 0.763 | 0.863 | 1.697 |
| IPI00908746.1 | cDNA FLJ51535, highly similar to Phosphatidylethanolamine-binding protein 1 | 2 | 1.215 | 1.080 | 1.494 | 0.992 | 0.979 | 1.399 |
| IPI00908881.3 | Glucose-6-phosphate isomerase | 21 | 1.390 | 1.889 | 4.093 | 1.235 | 1.632 | 0.778 |
| IPI00909239.1 | Isoform 2 of Alpha-actinin-1 | 2 | 0.959 | 1.185 | 3.107 | 0.847 | 0.983 | 0.761 |
| IPI00909530.1 | cDNA FLJ52843, highly similar to Histone H3.3 | 2 | 0.863 | 1.184 | 1.638 | 0.284 | 0.466 | 0.389 |
| IPI00909737.1 | cDNA FLJ55140, highly similar to SPARC-like protein 1 | 3 | 1.576 | 1.040 | 1.195 | 1.687 | 1.148 | 1.266 |
| IPI00910407.1 | Peptidyl-prolyl cis-trans isomerase | 4 | 1.257 | 1.380 | 2.526 | 1.134 | 1.306 | 1.122 |
| IPI00910819.2 | cDNA FLJ60194, highly similar to WW domain-binding protein 11 | 2 | 0.419 | 0.169 | 0.340 | 0.174 | 0.162 | 0.819 |
| IPI00911039.1 | cDNA FLJ54408, highly similar to Heat shock 70 kDa protein 1 | 24 | 1.286 | 1.230 | 2.977 | 0.919 | 1.177 | 1.015 |
| IPI00914858.1 | Isoform 3 of WD repeat- and FYVE domain-containing protein 4 | 1 | 0.710 | 0.782 | 2.267 | 0.797 | 0.708 | 0.764 |
| IPI00915959.2 | Uncharacterized protein | 1 | 1.027 | 0.847 | 1.058 | 0.822 | 1.078 | 1.278 |
| IPI00916434.1 | Anti-(ED-B) scFV (Fragment) | 22 | 1.451 | 1.070 | 1.836 | 1.129 | 1.270 | 1.745 |
| IPI00916818.1 | Phosphoglycerate kinase | 3 | 1.905 | 2.138 | 4.346 | 1.691 | 2.285 | 1.158 |
| IPI00918002.1 | Mucin 5AC, oligomeric mucus/gel-forming | 148 | 0.803 | 0.649 | 0.774 | 0.894 | 0.946 | 0.792 |
| IPI00921945.1 | cDNA FLJ57374 | 3 | 2.551 | 1.937 | 1.257 | 2.959 | 2.453 | 5.295 |
| IPI00922262.1 | cDNA FLJ56822, highly similar to Alpha-2-HS-glycoprotein | 3 | 1.453 | 1.413 | 2.569 | 1.226 | 1.486 | 0.917 |
| IPI00924751.1 | Protein | 2 | 6.044 | 5.591 | 1.745 | 9.960 | 7.333 | 6.159 |
| IPI00927887.1 | Histone H2A | 6 | 1.166 | 1.117 | 1.335 | 0.340 | 0.595 | 0.859 |
| IPI00929669.1 | Similar to Keratin 16 | 1 | 0.846 | 0.939 | 1.745 | 1.197 | 1.161 | 0.460 |
| IPI00930072.1 | Putative uncharacterized protein DKFZp686E23209 | 9 | 1.493 | 1.318 | 4.185 | 1.146 | 1.974 | 0.697 |
| IPI00930226.1 | cDNA FLJ57283, highly similar to Actin, cytoplasmic 2 | 144 | 1.614 | 2.170 | 5.681 | 1.448 | 1.915 | 0.897 |
| IPI00930442.1 | Putative uncharacterized protein DKFZp686M24218 | 10 | 1.357 | 1.336 | 3.471 | 0.849 | 1.304 | 0.710 |
| IPI00936444.2 | cDNA FLJ59081, highly similar to Mucin-5B | 375 | 0.767 | 0.618 | 0.731 | 0.872 | 0.943 | 0.744 |
| IPI00939521.1 | 10 kDa protein | 11 | 1.417 | 1.299 | 1.919 | 1.323 | 1.499 | 1.796 |
| IPI00940673.2 | cDNA FLJ36348 fis, clone THYMU2007025, highly similar to TRANSKETOLASE | 27 | 1.263 | 1.421 | 2.558 | 1.151 | 1.346 | 1.090 |
| IPI00942117.2 | cysteine-rich secretory protein 3 isoform 1 precursor | 3 | 1.602 | 1.679 | 1.851 | 2.268 | 1.658 | 1.236 |
| IPI00942257.3 | Uncharacterized protein | 8 | 0.831 | 1.298 | 2.436 | 1.171 | 1.063 | 0.668 |
| IPI00942979.1 | Transketolase | 56 | 1.799 | 2.383 | 4.969 | 1.448 | 2.125 | 0.778 |
| IPI00943894.1 | glycogen phosphorylase, liver form isoform 2 | 3 | 1.700 | 2.112 | 8.124 | 2.052 | 2.690 | 0.942 |
| IPI00945694.1 | Uncharacterized protein | 4 | 2.032 | 1.446 | 1.134 | 0.943 | 2.198 | 2.126 |
| IPI00946655.1 | Isoform 1 of Actin-related protein 3C | 6 | 1.390 | 1.496 | 5.005 | 1.472 | 2.156 | 0.949 |
| IPI00947240.1 | 24 kDa protein | 3 | 0.683 | 0.962 | 0.883 | 0.531 | 0.547 | 0.793 |
| IPI00964000.1 | Uncharacterized protein | 110 | 1.452 | 0.954 | 1.354 | 1.277 | 1.294 | 1.901 |
| IPI00965100.1 | Uncharacterized protein | 20 | 1.738 | 2.050 | 1.646 | 3.357 | 2.292 | 1.017 |
| IPI00965713.3 | fibrinogen beta chain isoform 2 preproprotein | 2 | 1.515 | 1.521 | 3.117 | 1.023 | 1.453 | 0.671 |
| IPI00966755.1 | Uncharacterized protein | 2 | 1.956 | 1.775 | 1.376 | 1.277 | 1.506 | 0.649 |
| IPI00967145.1 | Uncharacterized protein | 5 | 0.920 | 0.286 | 0.265 | 1.014 | 0.277 | 2.146 |
| IPI00968182.1 | Uncharacterized protein | 5 | 0.702 | 0.698 | 1.209 | 0.588 | 0.762 | 0.850 |
| IPI00969578.1 | Salivary proline-rich protein 2 (Fragment) | 3 | 0.384 | 0.298 | 0.467 | 0.428 | 0.296 | 2.190 |
| IPI00972963.1 | Lambda light chain of human immunoglobulin surface antigen-related protein (Fragment) | 28 | 1.623 | 1.250 | 2.236 | 1.587 | 1.700 | 2.028 |
| IPI00973998.1 | Uncharacterized protein | 4 | 0.855 | 1.217 | 1.587 | 0.311 | 0.383 | 0.315 |
| IPI00974112.1 | 22 kDa protein | 3 | 1.518 | 1.547 | 1.709 | 2.228 | 1.636 | 1.129 |
| IPI00974544.1 | Isoform SV of 14-3-3 protein epsilon | 4 | 1.198 | 1.054 | 1.576 | 0.983 | 1.235 | 0.878 |
| IPI00975690.1 | Vimentin variant 3 | 5 | 1.490 | 2.426 | 3.841 | 1.423 | 1.455 | 0.479 |
| IPI00975820.1 | Uncharacterized protein | 3 | 1.341 | 0.908 | 0.965 | 1.223 | 0.866 | 0.639 |
| IPI00976039.1 | Uncharacterized protein | 7 | 1.316 | 1.174 | 1.162 | 1.623 | 1.390 | 1.853 |
| IPI00976187.1 | Similar to Rheumatoid factor G9 heavy chain | 5 | 1.343 | 0.913 | 1.190 | 0.948 | 1.033 | 1.320 |
| IPI00976928.1 | Similar to Myosin-reactive immunoglobulin heavy chain variable region | 4 | 1.848 | 1.462 | 1.590 | 1.501 | 1.147 | 1.387 |
| IPI00977041.1 | Isoform 1 of Immunoglobulin lambda-like polypeptide 5 | 5 | 1.510 | 1.304 | 1.783 | 1.427 | 1.426 | 1.854 |
| IPI00977297.1 | Similar to VH4 heavy chain variable region precursor | 6 | 1.179 | 0.809 | 1.325 | 0.893 | 0.986 | 1.307 |
| IPI00977405.1 | Similar to Ig kappa chain V-III region VG precursor | 5 | 1.476 | 1.645 | 2.074 | 1.634 | 1.205 | 1.807 |
| IPI00977704.1 | Uncharacterized protein | 1 | 1.794 | 2.829 | 5.564 | 0.908 | 1.354 | 1.299 |
| IPI00977788.1 | Similar to Hepatitis B virus receptor binding protein | 6 | 1.015 | 0.952 | 1.480 | 1.052 | 0.982 | 1.314 |
| IPI00978315.1 | Conserved hypothetical protein | 2 | 0.987 | 0.913 | 1.992 | 0.499 | 0.712 | 0.576 |
| IPI00979837.1 | Conserved hypothetical protein | 3 | 1.470 | 1.866 | 3.592 | 1.451 | 1.808 | 0.801 |
| IPI00980674.1 | Uncharacterized protein | 11 | 2.724 | 4.406 | 9.159 | 0.884 | 1.734 | 1.214 |
| IPI00980807.1 | 5 kDa protein | 4 | 1.304 | 3.346 | 1.734 | 6.962 | 3.179 | 3.962 |
| IPI00981659.1 | Similar to Cold agglutinin FS-1 H-chain | 11 | 1.775 | 1.889 | 4.199 | 1.191 | 1.455 | 0.898 |
| IPI00982472.1 | Transaldolase | 19 | 1.276 | 1.416 | 3.039 | 1.011 | 1.238 | 0.745 |
| IPI00982588.1 | Protein | 2 | 1.404 | 1.095 | 1.125 | 1.587 | 1.258 | 2.002 |
| IPI00984004.1 | Similar to Ig kappa chain V-III region WOL | 1 | 1.345 | 1.206 | 1.794 | 1.449 | 1.176 | 1.690 |
| IPI00984370.1 | Uncharacterized protein | 1 | 1.681 | 2.137 | 2.999 | 2.333 | 2.090 | 2.109 |
| IPI00984640.1 | Similar to Immunglobulin heavy chain variable region | 15 | 2.300 | 2.083 | 2.111 | 1.618 | 1.775 | 1.900 |
| IPI00984835.1 | 16 kDa protein | 2 | 4.667 | 1.241 | 0.485 | 3.896 | 1.002 | 12.522 |
| IPI00985334.2 | titin isoform N2-B | 3 | 1.057 | 1.108 | 2.004 | 0.654 | 1.002 | 0.621 |
| IPI00985505.1 | Uncharacterized protein | 3 | 0.795 | 1.052 | 0.653 | 1.359 | 0.731 | 2.521 |
| IPI01009389.1 | DNA methyltransferase | 3 | 4.745 | 3.371 | 0.401 | 6.191 | 4.063 | 12.096 |
| IPI01009456.2 | 34 kDa protein | 3 | 1.200 | 1.522 | 3.292 | 1.059 | 1.495 | 0.874 |
| IPI01009563.1 | Adenylyl cyclase-associated protein | 3 | 1.488 | 1.598 | 4.019 | 1.105 | 1.534 | 0.762 |
| IPI01010684.1 | Uncharacterized protein | 7 | 0.855 | 0.667 | 1.161 | 1.015 | 0.862 | 2.082 |
| IPI01011090.1 | Uncharacterized protein | 2 | 1.759 | 2.275 | 2.702 | 1.622 | 1.984 | 1.655 |
| IPI01011344.1 | Uncharacterized protein | 35 | 1.710 | 2.297 | 4.424 | 1.556 | 2.008 | 0.946 |
| IPI01011676.1 | Uncharacterized protein | 4 | 1.390 | 1.652 | 2.651 | 0.848 | 1.032 | 1.002 |
| IPI01011820.1 | Uncharacterized protein | 4 | 1.286 | 1.318 | 2.605 | 1.057 | 1.352 | 0.779 |
| IPI01011970.1 | 6-phosphogluconate dehydrogenase, decarboxylating | 12 | 1.270 | 1.514 | 2.734 | 1.340 | 1.478 | 0.906 |
| IPI01012346.1 | Uncharacterized protein | 2 | 1.617 | 2.046 | 5.188 | 1.135 | 1.631 | 0.652 |
| IPI01012426.1 | Uncharacterized protein | 2 | 1.070 | 1.154 | 2.030 | 0.831 | 0.852 | 0.952 |
| IPI01012504.1 | 6-phosphogluconate dehydrogenase, decarboxylating | 38 | 1.359 | 1.542 | 3.026 | 1.406 | 1.599 | 0.918 |
| IPI01012528.1 | Uncharacterized protein | 15 | 1.690 | 1.785 | 3.294 | 1.624 | 1.882 | 1.142 |
| IPI01013019.1 | Airway lactoperoxidase | 24 | 0.882 | 0.733 | 1.101 | 1.167 | 0.906 | 2.216 |
| IPI01013112.1 | Uncharacterized protein | 2 | 1.502 | 1.616 | 3.136 | 1.163 | 1.581 | 0.786 |
| IPI01013441.1 | Uncharacterized protein | 37 | 1.478 | 1.614 | 3.728 | 1.113 | 1.452 | 0.624 |
| IPI01013537.1 | Uncharacterized protein | 8 | 1.128 | 0.999 | 1.396 | 1.072 | 0.980 | 1.148 |
| IPI01013543.1 | Triosephosphate isomerase | 3 | 0.553 | 0.639 | 1.933 | 0.614 | 0.563 | 0.726 |
| IPI01014238.1 | cDNA FLJ53963, highly similar to Leukocyte elastase inhibitor | 3 | 1.093 | 0.902 | 0.940 | 1.238 | 1.013 | 1.197 |
| IPI01014975.1 | Uncharacterized protein | 4 | 1.298 | 1.759 | 6.183 | 1.213 | 1.747 | 0.620 |
| IPI01015050.2 | Uncharacterized protein | 10 | 1.075 | 1.221 | 3.161 | 0.882 | 1.159 | 0.708 |
| IPI01015184.1 | Uncharacterized protein | 2 | 1.649 | 1.436 | 1.903 | 1.081 | 1.226 | 1.151 |
| IPI01015504.1 | Uncharacterized protein | 5 | 1.726 | 1.551 | 2.266 | 1.769 | 1.763 | 2.780 |
| IPI01015565.1 | Uncharacterized protein | 3 | 2.337 | 2.119 | 2.126 | 1.706 | 1.583 | 1.924 |
| IPI01015921.1 | cDNA FLJ55361, highly similar to Nucleolar protein 11 | 5 | 3.253 | 6.211 | 2.964 | 1.562 | 1.872 | 2.294 |
| IPI01018060.1 | Ig lambda-3 chain C regions | 23 | 1.272 | 0.992 | 1.609 | 1.476 | 1.335 | 1.720 |
| IPI01019128.1 | Uncharacterized protein | 1 | 1.461 | 1.790 | 1.194 | 0.640 | 0.973 | 1.539 |
| IPI01021118.1 | Uncharacterized protein | 1 | 0.671 | 0.503 | 0.728 | 0.286 | 0.369 | 1.274 |
| IPI01022175.1 | cDNA FLJ55805, highly similar to Keratin, type II cytoskeletal 4 | 13 | 0.751 | 3.532 | 8.674 | 0.944 | 0.728 | 0.836 |
| IPI01022408.1 | Uncharacterized protein | 3 | 1.609 | 1.588 | 2.160 | 1.128 | 1.363 | 1.526 |
| IPI01022662.1 | Uncharacterized protein | 3 | 1.561 | 1.720 | 1.533 | 1.291 | 1.122 | 1.085 |
| IPI01023021.1 | Uncharacterized protein | 2 | 2.702 | 1.781 | 2.674 | 1.532 | 1.735 | 1.014 |
| IPI01024806.1 | Alpha-actinin 1 | 5 | 1.098 | 1.431 | 3.435 | 1.031 | 1.276 | 0.762 |
| IPI01026033.1 | 9 kDa protein | 2 | 0.604 | 0.402 | 0.671 | 0.552 | 0.550 | 1.382 |
| IPI01026288.1 | Uncharacterized protein | 14 | 1.097 | 1.071 | 1.045 | 1.123 | 1.014 | 2.762 |
| O83224 | 50S ribosomal protein L22 OS=Treponema pallidum (strain Nichols) GN=rpIV PE=3 SV=1 - [RL22_TREPA] | 2 | 2.132 | 3.140 | 3.922 | 1.528 | 2.328 | 1.160 |
| O83465 | Uncharacterized protein TP_0451 OS=Treponema pallidum (strain Nichols) GN=TP_0451 PE=4 SV=1 - [Y451_TREPA] | 2 | 4.618 | 3.313 | 1.304 | 3.726 | 3.516 | 5.718 |
| O83773 | Uncharacterized protein TP_0795 OS=Treponema pallidum (strain Nichols) GN=TP_0795 PE=4 SV=1 - [Y795_TREPA] | 2 | 4.450 | 9.222 | 10.821 | 1.969 | 4.533 | 0.810 |
| P24366 | Phosphocarrier protein HPr OS = Streptococcus salivarius GN=ptsH PE=1 SV=2 - [PTHP_STRSL] | 3 | 0.735 | 0.259 | 0.797 | 0.316 | 0.262 | 1.029 |
| P96119 | Zinc transport system membrane protein troD OS=Treponema pallidum (strain Nichols) GN=troD PE=3 SV=1 - [TROD_TREPA] | 3 | 1.209 | 1.223 | 1.320 | 1.302 | 2.272 | 1.126 |
| Q9AIM3 | 3-isopropylmalate dehydratase large subunit (Fragment) OS=Streptococcus gordonii GN=leuC PE=3 SV=1 - [LEUC_STRGN] | 2 | 2.397 | 1.049 | 0.615 | 1.752 | 0.661 | 5.701 |

**SUPPLEMENTARY TABLE 6**

| CLUSTER 2 | | |
|---|---|---|
| Protein # 165: | IPI00748022.2 | Actin-like protein (Fragment) |

**SUPPLEMENTARY TABLE 7**

| CLUSTER 1B | | |
|---|---|---|
| Protein # 19: | IPI00012199.1 | Coiled-coil domain-containing protein 86 |
| Protein # 58: | IPI00060800.5 | Zymogen granule protein 16 homolog B |
| Protein # 73: | IPI00216835.2 | Isoform 2 of NADPH oxidase activator 1 |
| Protein # 94: | IPI00299078.1 | Salivary acidic proline-rich phosphoprotein 1/2 |
| Protein # 108: | IPI00383627.1 | Pituitary tumor transforming gene protein |
| Protein # 110: | IPI00384251.1 | Isoform 2 of Guanine nucleotide exchange factor for Rab-3A |
| Protein # 111: | IPI00384382.1 | AngRem52 |
| Protein # 122: | IPI00414909.1 | Alpha-N-acetylgalactosaminidase |
| Protein # 163: | IPI00742775.1 | Bardet-Biedl syndrome 10 protein |
| Protein # 221: | IPI00921945.1 | cDNA FLJ57374 |
| Protein # 270: | IPI00984835.1 | 16 kDa protein |
| Protein # 273: | IPI00985334.2 | titin isoform N2-B |
| Protein # 297: | IPI01015921.1 | cDNA FLJ55361, highly similar to Nucleolar protein 11 |
| Protein # 309: | O83465 | Uncharacterized protein TP_0451 OS=Treponema pallidum (strain Nichols) GN=TP_0451 PE=4 SV=1 - [Y451_TREPA] |

| CLUSTER 1D | | |
|---|---|---|
| Protein # 212: | IPI00909737.1 | cDNA FLJ55140, highly similar to SPARC-like protein 1 |

**SUPPLEMENTARY TABLE 8**

| CLUSTER 1A4 | | |
|---|---|---|
| Protein # 21: | IPI00012796.1 | Glutamate decarboxylase 2 |
| Protein # 300: | IPI01021118.1 | Uncharacterized protein |
| Protein # 311: | P24366 | Phosphocarrier protein HPr OS=Streptococcus salivarius GN=ptsH PE=1 SV=2 - [PTHP_STRSL] |

| CLUSTER 1A5 | | |
|---|---|---|
| Protein # 245: | IPI00969578.1 | Salivary proline-rich protein 2 (Fragment) |

**SUPPLEMENTARY TABLE 9**

| CLUSTER 1C2 | | |
|---|---|---|
| Protein # 26: | IPI00016347.5 | Isoform 3 of Uncharacterized protein C2orf54 |
| Protein# 107: | IPI00377122.4 | Isoform 2 of WD repeat-containing protein KIAA1875 |
| Protein# 120: | IPI00410714.5 | Hemoglobin subunit alpha |
| Protein# 135: | IPI00473011.3 | Hemoglobin subunit delta |
| Protein# 157: | IPI00654755.3 | Hemoglobin subunit beta |
| Protein # 262: | IPI00980674.1 | Uncharacterized protein |
| Protein # 310: | O83773 | Uncharacterized protein TP_0795 OS=Treponema pallidum (strain Nichols) GN=TP_0795 PE=4 SV=1 - [Y795_TREPA] |

**SUPPLEMENTAL TABLE 10**

| CLUSTER 1A1b | | |
|---|---|---|
| Protein# 25: | IPI00013895.1 | Protein S100-A11 |
| Protein # 57: | IPI00037070.3 | Uncharacterized protein |
| Protein # 132: | IPI00465436.4 | Catalase |
| Protein # 206: | IPI00903245.1 | cDNA FLJ44586 fis, clone ASTRO2015162, highly similar to Choline transporter-like protein 2 |
| Protein # 271: | IPI00985334.2 | titin isoform N2-B |

**SUPPLEMENTAL TABLE 11**

| Description | Accession | gene | Description |
|---|---|---|---|
| Beta-actin-like protein 2 | IPI00003269.1 | ACTBL2 | Beta-actin-like protein 2 |
| Histone H2B type 2-E | IPI00003935.6 | HIST2H2BE | Histone H2B type 2-E |
| Neutrophil defensin 1 | IPI00005721.1 | DEFA1 | Neutrophil defensin 1 |
| Protein S100-A8 | IPI00007047.1 | S100A8 | Protein S100-A8 |
| Arylsulfatase F | IPI00008405.5 | ARSF | Arylsulfatase F |
| Epithelial membrane protein 2 | IPI00008895.1 | EMP2 | Epithelial membrane protein 2 |
| Plastin-2 | IPI00010471.6 | LCP1 | Plastin-2 |
| Isoform 1 of 14-3-3 protein sigma | IPI00013890.2 | SFN | Isoform 1 of 14-3-3 protein sigma |
| Protein S100-A11 | IPI00013895.1 | S100A11 | Protein S100-A11 |
| Protein S100-P | IPI00017526.1 | S100P | Protein S100-P |
| Lysozyme C | IPI00019038.1 | LYZ | Lysozyme C |
| Isoform 1 of Myosin-9 | IPI00019502.3 | MYH9 | Isoform 1 of Myosin-9 |
| Alpha-1-acid glycoprotein 2 | IPI00020091.1 | ORM2 | Alpha-1-acid glycoprotein 2 |
| 14-3-3 protein zeta/delta | IPI00021263.3 | YWHAZ | 14-3-3 protein zeta/delta |
| Cystatin-B | IPI00021828.1 | CSTB | Cystatin-B |
| Apolipoprotein A-I | IPI00021841.1 | APOA1 | Apolipoprotein A-I |
| Alpha-1-acid glycoprotein 1 | IPI00022429.3 | ORM1 | Alpha-1-acid glycoprotein 1 |
| Serotransferrin | IPI00022463.2 | TF | Serotransferrin |
| Hemopexin | IPI00022488.1 | HPX | Hemopexin |
| Prolactin-inducible protein | IPI00022974.1 | PIP | Prolactin-inducible protein |
| Protein S100-A9 | IPI00027462.1 | S100A9 | Protein S100-A9 |
| Protein S100-A6 | IPI00027463.1 | S100A6 | Protein S100-A6 |
| Matrix metalloproteinase-9 | IPI00027509.5 | MMP9 | Matrix metalloproteinase-9 |
| Neutrophil elastase | IPI00027769.1 | ELANE | Neutrophil elastase |
| Cathepsin G | IPI00028064.1 | CTSG | Cathepsin G |
| Cystatin-S | IPI00032294.1 | CST4 | Cystatin-S |
| Uncharacterized protein | IPI00037070.3 | HSPA8 | Uncharacterized protein |
| Isoform 4 of Interleukin-1 receptor antagonist protein | IPI00174541.1 | IL1RN | Isoform 4 of Interleukin-1 receptor antagonist protein |
| Thymosin beta-4-like protein 3 | IPI00180240.2 | TMSL3 | Thymosin beta-4-like protein 3 |
| Profilin-1 | IPI00216691.5 | PFN1 | Profilin-1 |
| Keratin, type I cytoskeletal 16 | IPI00217963.3 | KRT16 | Keratin, type I cytoskeletal 16 |
| Protein S100-A12 | IPI00218131.3 | S100A12 | Protein S100-A12 |
| Annexin A1 | IPI00218918.5 | ANXA1 | Annexin A1 |
| Glutathione S-transferase P | IPI00219757.13 | GSTP1 | Glutathione S-transferase P |
| Keratin, type II cytoskeletal 1 | IPI00220327.4 | KRT1 | Keratin, type II cytoskeletal 1 |
| Isoform H14 of Myeloperoxidase | IPI00236554.1 | MPO | Isoform H14 of Myeloperoxidase |
| Keratin, type I cytoskeletal 15 | IPI00290077.3 | KRT15 | Keratin, type I cytoskeletal 15 |
| Fibrinogen beta chain | IPI00298497.3 | FGB | Fibrinogen beta chain |
| Salivary acidic proline-rich phosphoprotein 1/2 | IPI00299078.1 | PRH1 | Salivary acidic proline-rich phosphoprotein 1/2 |
| Isoform 1 of Neutrophil gelatinase-associated lipocalin | IPI00299547.4 | LCN2 | Isoform 1 of Neutrophil gelatinase-associated lipocalin |
| Cystatin-SN | IPI00305477.6 | CST1 | Cystatin-SN |
| Keratin, type I cytoskeletal 14 | IPI00384444.6 | KRT14 | Keratin, type I cytoskeletal 14 |
| Putative uncharacterized protein DKFZp686I04196 (Fragment) | IPI00399007.7 | IGHG2 | Putative uncharacterized protein DKFZp686I04196 (Fragment) |
| Hemoglobin subunit alpha | IPI00410714.5 | HBA1 | Hemoglobin subunit alpha |
| Peptidyl-prolyl cis-trans isomerase A | IPI00419585.9 | PPIA | Peptidyl-prolyl cis-trans isomerase A |
| Putative uncharacterized protein DKFZp686G21220 (Fragment) | IPI00423460.3 | IGHA1 | Putative uncharacterized protein DKFZp686G21220 (Fragment) |
| 31 kDa protein | IPI00431645.2 | HPR | 31 kDa protein |
| 44 kDa protein | IPI00448925.6 | IGHG1 | 44 kDa protein |
| Histone H4 | IPI00453473.6 | HIST4H4 | Histone H4 |
| Isoform alpha-enolase of Alpha-enolase | IPI00465248.5 | ENO1 | Isoform alpha-enolase of Alpha-enolase |
| Fructose-bisphosphate aldolase A | IPI00465439.5 | ALDOA | Fructose-bisphosphate aldolase A |
| haptoglobin isoform 2 preproprotein | IPI00478493.3 | HPR | haptoglobin isoform 2 preproprotein |
| Isoform M2 of Pyruvate kinase isozymes M1/M2 | IPI00479186.7 | PKM2 | Isoform M2 of Pyruvate kinase isozymes M1/M2 |
| Uncharacterized protein | IPI00552768.1 | TXN | Uncharacterized protein |
| Haptoglobin | IPI00641737.2 | HP | Haptoglobin |
| Hemoglobin subunit beta | IPI00654755.3 | HBB | Hemoglobin subunit beta |
| Isoform 1 of Serum albumin | IPI00745872.2 | ALB | Isoform 1 of Serum albumin |
| Actin-like protein (Fragment) | IPI00748022.2 | LOC727848 | Actin-like protein (Fragment) |
| Isoform Cytoplasmic+peroxisomal of Peroxiredoxin-5, mitochondrial | IPI00759663.1 | PRDX5 | Isoform Cytoplasmic+peroxisomal of Peroxiredoxin-5, mitochondrial |
| Complement C3 (Fragment) | IPI00783987.2 | C3 | Complement C3 (Fragment) |
| Putative uncharacterized protein DKFZp686L19235 | IPI00784950.1 | IGHA2 | Putative uncharacterized protein DKFZp686L19235 |
| IGK@ protein | IPI00784985.1 | IGK@ | IGK@ protein |
| Isoform 1 of Triosephosphate isomerase | IPI00797270.4 | TPI1P1 | Isoform 1 of Triosephosphate isomerase |
| FGB protein (Fragment) | IPI00816687.1 | FGB | FGB protein (Fragment) |
| Uncharacterized protein | IPI00853525.1 | APOA1 | Uncharacterized protein |
| Protein | IPI00879437.1 | P4HB | Protein |
| Uncharacterized protein | IPI00893981.1 | ACTB | Uncharacterized protein |
| FGA protein (Fragment) | IPI00902755.1 | FGA | FGA protein (Fragment) |
| cDNA FLJ36533 fis, clone TRACH2004428, highly similar to Lactotransferrin (Fragment) | IPI00903112.1 | LTF | cDNA FLJ36533 fis, clone TRACH2004428, highly similar to Lactotransferrin (Fragment) |
| cDNA FLJ51275 | IPI00908402.1 | CRNN | cDNA FLJ51275 |
| Glucose-6-phosphate isomerase | IPI00908881.3 | GPI | Glucose-6-phosphate isomerase |
| cDNA FLJ52843, highly similar to Histone H3.3 | IPI00909530.1 | LOC644914 | cDNA FLJ52843, highly similar to Histone H3.3 |
| Peptidyl-prolyl cis-trans isomerase | IPI00910407.1 | PPIA | Peptidyl-prolyl cis-trans isomerase |
| cDNA FLJ54408, highly similar to Heat shock 70 kDa protein 1 | IPI00911039.1 | HSPA1A | cDNA FLJ54408, highly similar to Heat shock 70 kDa protein 1 |
| Histone H2A | IPI00927887.1 | H2AFV | Histone H2A |
| Putative uncharacterized protein DKFZp686M24218 | IPI00930442.1 | IGHG4 | Putative uncharacterized protein DKFZp686M24218 |
| 10 kDa protein | IPI00939521.1 | | 10 kDa protein |
| cDNA FLJ36348 fis, clone THYMU2007025, highly similar to TRANSKETOLASE | IPI00940673.2 | TKT | cDNA FLJ6348 fis, clone THYMU2007025, highly similar to TRANSKETOLASE |
| Isoform 1 of Actin-related protein 3C | IPI00946655.1 | ACTR3C | Isoform 1 of Actin-related protein 3C |
| fibrinogen beta chain isoform 2 preproprotein | IPI00965713.3 | FGB | fibrinogen beta chain isoform 2 preproprotein |
| Lambda light chain of human immunoglobulin surface antigen-related protein (Fragment) | IPI00972963.1 | IgLC-rG | Lambda light chain of human immunoglobulin surface antigen-related protein (Fragment) |
| Vimentin variant 3 | IPI00975690.1 | VIM | Vimentin variant 3 |
| Uncharacterized protein | IPI00980674.1 | CA1 | Uncharacterized protein |
| Similar to Cold agglutinin FS-1 H-chain | IPI00981659.1 | IGH@ | Similar to Cold agglutinin FS-1 H-chain |
| Transaldolase | IPI00982472.1 | TALDO1 | Transaldolase |
| titin isoform N2-B | IPI00985334.2 | TTN | titin isoform N2-B |
| Uncharacterized protein | IPI01011344.1 | ACTG1 | Uncharacterized protein |
| 6-phosphogluconate dehydrogenase, decarboxylating | IPI01011970.1 | PGD | 6-phosphogluconate dehydrogenase, decarboxylating |
| Uncharacterized protein | IPI01013112.1 | ARHGDIB | Uncharacterized protein |
| Uncharacterized protein | IPI01013441.1 | PRTN3 | Uncharacterized protein |
| Triosephosphate isomerase | IPI01013543.1 | TPI1 | Triosephosphate isomerase |
| Uncharacterized protein | IPI01014975.1 | TLN1 | Uncharacterized protein |
| Uncharacterized protein | IPI01015050.2 | GSN | Uncharacterized protein |
| Ig lambda-3 chain C regions | IPI01018060.1 | IGLC3 | Ig lambda-3 chain C regions |
| cDNA FLJ55805, highly similar to Keratin, type II cytoskeletal 4 | IPI01022175.1 | KRT4 | cDNA FLJ55805, highly similar to Keratin, type II cytoskeletal 4 |

**SUPPLEMENTAL TABLE 12**

| **GCF** | **Biological process** | **Count (genes)** | **P-value** |
|---|---|---|---|
| | cytoskeleton organization | 29 | 1.90E-12 |
| | glucose catabolic process | 12 | 1.60E-10 |
| | actin cytoskeleton organization | 19 | 7.50E-10 |
| | hexose catabolic process | 12 | 1.20E-09 |
| | monosaccharide catabolic process | 12 | 1.60E-09 |
| | actin filament-based process | 19 | 2.10E-09 |
| | alcohol catabolic process | 12 | 6.70E-09 |
| | glycolysis | 10 | 7.20E-09 |
| | organelle organization | 44 | 1.10E-08 |
| | cellular carbohydrate catabolic process | 12 | 1.10E-08 |
| | defense response | 28 | 2.30E-08 |
| | ectoderm development | 16 | 4.40E-08 |
| | response to stimulus | 79 | 6.40E-08 |
| | cellular component organization | 63 | 7.00E-08 |
| | carbohydrate catabolic process | 12 | 1.60E-07 |
| | cellular component assembly | 31 | 1.10E-06 |
| | response to stress | 46 | 1.20E-06 |
| | tissue development | 26 | 1.40E-06 |
| | response to external stimulus | 31 | 1.90E-06 |

| **Saliva** | **Biological process** | **Count (genes)** | **P-value** |
|---|---|---|---|
| | defense response | 32 | 1.60E-10 |
| | glucose catabolic process | 11 | 4.40E-09 |
| | response to stimulus | 83 | 1.50E-08 |
| | cellular carbohydrate catabolic process | 12 | 1.70E-08 |
| | carbohydrate catabolic process | 13 | 2.40E-08 |
| | hexose catabolic process | 11 | 2.50E-08 |
| | monosaccharide catabolic process | 11 | 3.30E-08 |
| | response to stress | 50 | 8.60E-08 |
| | response to inorganic substance | 16 | 1.10E-07 |
| | alcohol catabolic process | 11 | 1.20E-07 |
| | response to wounding | 25 | 1.70E-07 |
| | glycolysis | 9 | 1.80E-07 |
| | inflammatory response | 19 | 3.70E-07 |
| | response to external stimulus | 33 | 4.80E-07 |
| | tissue development | 27 | 8.60E-07 |
| | actin cytoskeleton organization | 15 | 2.10E-06 |
| | defense response to bacterium | 11 | 2.50E-06 |
| | ectoderm development | 14 | 2.80E-06 |
| | immune system process | 33 | 3.20E-06 |

## Claims

1. A method for diagnosing the status of periodontitis disease in a patient, comprising:
providing at least one of a gingival crevicular fluid (GCF) sample and a saliva sample; and
determining the expression levels of a set of protein biomarkers comprising haemoglobin alpha chain, haemoglobin beta chain, carbonic anhydrase 1 and plastin-1 in the sample, to diagnose whether the patient's periodontis status is healthy, gingivitis, mild periodontitis or severe periodontitis.

2. The method according to claim 1, wherein the set of protein biomarkers distinguishes between a gingivitis state and a periodontitis state.

3. The method according to claim 1, wherein the set of protein biomarkers distinguishes between a periodontal health and a disease state.

4. The method according to claim 1, wherein the set of protein biomarkers selected distinguishes between a mild periodontitis state and a severe periodontitis state.

5. A kit for diagnosing the status of periodontitis disease, comprising reagents necessary to identify a set of protein biomarkers selected to distinguish between gingivitis and stages of periodontitis, wherein the set of protein biomarkers comprises haemoglobin alpha chain, haemoglobin beta chain, carbonic anhydrase 1 and plastin 1.

6. The kit according to claim 5, wherein the kit diagnoses gingivitis or mild periodontitis, and the set of protein biomarkers further includes at least one protein biomarker selected from coiled-coil domain-containing protein 86, zymogen granule protein 16 homolog B, isoform 2 of NADPH oxidase activator 1, salivary acidic proline-rich phosphoprotein 1/2, pituitary tumor transforming gene protein, isoform 2 of Guanine nucleotide exchange factor for Rab-3A, AngRem52, Alpha-N-acetylgalactosaminidase, Bardet-Biedl syndrome 10 protein, cDNA FLJ57374, 16 kDa protein, titin isoform N2-B, uncharacterized protein TP_0451 OS=Treponema pallidum (strain Nichols) GN=TP_0451 PE=4 SV=1 - [Y451_TREPA], cDNA FLJ55140, Glutamate decarboxylase 2, protein having International Protein Index or "IPI" # IPI01021118.1, Phosphocarrier protein HPr OS=Streptococcus salivarius GN=ptsH PE=1 SV=2 - [PTHP_STRSL], and salivary proline-rich protein 2.
